# EUROPÄISCHE PATENTSCHRIFT

(11) **EP 1 857 555 B1**
(45) Veröffentlichungstag und Bekanntmachung des Hinweises auf die Patenterteilung: **31.12.2008**
(21) Anmeldenummer: 07013576.9
(22) Anmeldetag: 01.10.2004
(51) Int. Cl.: C12N 15/63, A61K 48/00, C12N 15/10

(54) **Verfahren zur Herstellung eines Zell- und/oder Gewebe- und/oder Krankheitsphasen-spezifischen Arzneimittels**
Method for the production of a cell and/or tissue disease phase specific treatment
Méthode pour la production d'un médicament spécifique pour de phase de cellules et/ou de maladie de tissu

(30) Priorität: 02.10.2003 DE 10346487
(43) Veröffentlichungstag der Anmeldung: 21.11.2007
(62) Teilanmeldung aus: 04802618.1
(73) Patentinhaber: STERNA BIOLOGICALS GmbH & Co. KG, 35043 Marburg (DE)
(72) Erfinder: Sel, Serdar, Dr., 35043 Marburg (DE); Renz, Harald, Prof. Dr., 35043 Marburg-Cappel (DE)
(74) Vertreter: Meyer-Dulheuer, Karl-Hermann

(56) Entgegenhaltungen:
- WO-A-00/42173
- WO-A-01/11023
- SANTORDO S W ET AL: "A GENERAL PURPOSE RNA-CLEAVING DNA ENZYME" PROCEEDINGS OF THE NATIONAL ACADEMY OF SCIENCES OF USA, NATIONAL ACADEMY OF SCIENCE. WASHINGTON, US, Bd. 94, April 1997 (1997-04), Seiten 4262-4266, XP001009844 ISSN: 0027-8424
- SUN L Q ET AL: "Catalytic nucleic acids: From lab to applications" September 2000 (2000-09), PHARMACOLOGICAL REVIEWS, WILLIAMS AND WILKINS INC., BALTIMORE, MD,, US, PAGE(S) 325-347 , XP002272275 ISSN: 0031-6997 * Seite 330 - Seite 337 *

### SEQUENCE LISTING

<110> Philipps-Universität Marburg
<120> verfahren zur Herstellung eines zell- und/oder Gewebe- und/oder Krankheitsphasen-spezifischen Arzneimittels
<130> unknown
<140> PCT/DE2004/002197
   <141> 2004-10-01
<150> DE 10346487.5
   <151> 2003-10-02
<160> 80
<170> PatentIn version 3.3
<210> 1
   <211> 33
   <212> DNA
   <213> Homo sapiens
<220>
   <221> td1 DNAzyme against T-bet mRNA
   <222> (1)..(33)
<400> 1
   tggcttctag gctagctaca acgagccctc gtc 33
<210> 2
   <211> 33
   <212> DNA
   <213> Homo sapiens
<220>
   <221> td2 DNAzyme against T-bet mRNA
   <222> (1)..(33)
<400> 2
   gggctctgag gctagctaca acgagcctgg ctt 33
<210> 3
   <211> 33
   <212> DNA
   <213> Homo sapiens
<220>
   <221> td3 DNAzyme against T-bet mRNA
   <222> (1)..(33)
<400> 3
   gggaccccag gctagctaca acgacggagc ccg 33
<210> 4
   <211> 33
   <212> DNA
   <213> Homo sapiens
<220>
   <221> td4 DNAzyme against T-bet mRNA
   <222> (1)..(33)
<400> 4
   ggtgggggag gctagctaca acgacccacc gga 33
<210> 5
   <211> 33
   <212> DNA
   <213> Homo sapiens
<220>
   <221> td5 DNAzyme against T-bet mRNA
   <222> (1)..(33)
<400> 5
   ggcgggggag gctagctaca acgaccgagg gcc 33
<210> 6
   <211> 33
   <212> DNA
   <213> Homo sapiens
<220>
   <221> td6 DNAzyme against T-bet mRNA
   <222> (1)..(33)
<400> 6
   gggctgggag gctagctaca acgagggcag gga 33
<210> 7
   <211> 33
   <212> DNA
   <213> Homo sapiens
<220>
   <221> td7 DNAzyme against T-bet mRNA
   <222> (1)..(33)
<400> 7
   cgtcgaggag gctagctaca acgaccgccc ctc 33
<210> 8
   <211> 33
   <212> DNA
   <213> Homo sapiens
<220>
   <221> td8 DNAzyme against T-bet mRNA
   <222> (1)..(33)
<400> 8
   gggctggcag gctagctaca acgacttccc gta 33
<210> 9
   <211> 33
   <212> DNA
   <213> Homo sapiens
<220>
   <221> td9 DNAzyme against T-bet mRNA
   <222> (1)..(33)
<400> 9
   cgatgcccag gctagctaca acgaccgggg cgg 33
<210> 10
   <211> 33
   <212> DNA
   <213> Homo sapiens
<220> .
   <221> td10 DNAzyme against T-bet mRNA
   <222> (1)..(33)
<400> 10
   gctccacgag gctagctaca acgagcccat ccg 33
<210> 11
   <211> 33
   <212> DNA
   <213> Homo sapiens
<220>
   <221> td11 DNAzyme against T-bet mRNA
   <222> (1)..(33)
<400> 11
   ccggctccag gctagctaca acgagatgcc cat 33
<210> 12
   <211> 33
   <212> DNA
   <213> Homo sapiens
<220>
   <221> td12 DNAzyme against T-bet mRNA
   <222> (1)..(33)
<400> 12
   tctccgcaag gctagctaca acgaccggct cca 33
<210> 13
   <211> 33
   <212> DNA
   <213> Homo sapiens
<220>
   <221> td13 DNAzyme against T-bet mRNA
   <222> (1)..(33)
<400> 13
   ccgtcagcag gctagctaca acgagtctcc gca 33
<210> 14
   <211> 33
   <212> DNA
   <213> Homo sapiens
<220>
   <221> td14 DNAzyme against T-bet mRNA
   <222> (1)..(33)
<400> 14
   tccccggcag gctagctaca acgacggctc ggt 33
<210> 15
   <211> 33
   <212> DNA
   <213> Homo sapiens
<220>
   <221> td15 DNAzyme against T-bet mRNA
   <222> (1)..(33)
<400> 15
   cccccgcgag gctagctaca acgagctcgt ccg 33
<210> 16
   <211> 33
   <212> DNA
   <213> Homo sapiens
<220>
   <221> td16 DNAzyme against T-bet mRNA
   <222> (1)..(33)
<400> 16
   gtagggagag gctagctaca acgacccagg ctg 33
<210> 17
   <211> 33
   <212> DNA
   <213> Homo sapiens
<220>
   <221> td17 DNAzyme against T-bet mRNA
   <222> (1)..(33)
<400> 17
   gggcgggcag gctagctaca acgacaaggc gcc 33
<210> 18
   <211> 33
   <212> DNA
   <213> Homo sapiens
<220>
   <221> td18 DNAzyme against T-bet mRNA
   <222> (1)..(33)
<400> 18
   cgggaaggag gctagctaca acgatcgccc gcg 33
<210> 19
   <211> 33
   <212> DNA
   <213> Homo sapiens
<220>
   <221> td19 DNAzyme against T-bet mRNA
   <222> (1)..(33)
<400> 19
   tagtcctcag gctagctaca acgagcggcc ccg 33
<210> 20
   <211> 33
   <212> DNA
   <213> Homo sapiens
<220>
   <221> td20 DNAzyme against T-bet mRNA
   <222> (1)..(33)
<400> 20
   tccccgacag gctagctaca acgactccag tcc 33
<210> 21
   <211> 33
   <212> DNA
   <213> Homo sapiens
<220>
   <221> td21 DNAzyme against T-bet mRNA
   <222> (1)..(33)
<400> 21
   tttccccgag gctagctaca acgaacctcc agt 33
<210> 22
   <211> 33
   <212> DNA
   <213> Homo sapiens
<220>
   <221> td22 DNAzyme against T-bet mRNA
   <222> (1)..(33)
<400> 22
   tgagcgcgag gctagctaca acgacctcag ttt 33
<210> 23
   <211> 33
   <212> DNA
   <213> Homo sapiens
<220>
   <221> td23 DNAzyme against T-bet mRNA
   <222> (1)..(33)
<400> 23
   ggaccacaag gctagctaca acgaaggtgg ttg 33
<210> 24
   <211> 33
   <212> DNA
   <213> Homo sapiens
<220>
   <221> td24 DNAzyme against T-bet mRNA
   <222> (1)..(33)
<400> 24
   cttggaccag gctagctaca acgaaacagg tgg 33
<210> 25
   <211> 33
   <212> DNA
   <213> Homo sapiens
<220>
   <221> td25 DNAzyme against T-bet mRNA
   <222> (1)..(33)
<400> 25
   aaacttggag gctagctaca acgacacaac agg 33
<210> 26
   <211> 33
   <212> DNA
   <213> Homo sapiens
<220>
   <221> td26 DNAzyme against T-bet mRNA
   <222> (1)..(33)
<400> 26
   ctgattaaag gctagctaca acgattggac cac 33
<210> 27
   <211> 33
   <212> DNA
   <213> Homo sapiens
<220>
   <221> td27 DNAzyme against T-bet mRNA
   <222> (1)..(33)
<400> 27
   tggtgctgag gctagctaca acgataaact tgg 33
<210> 28
   <211> 33
   <212> DNA
   <213> Homo sapiens
<220>
   <221> td28 DNAzyme against T-bet mRNA
   <222> (1)..(33)
<400> 28
   tgatgatcag gctagctaca acgactctgt ctg 33
<210> 29
   <211> 33
   <212> DNA
   <213> Homo sapiens
<220>
   <221> td29 DNAzyme against T-bet mRNA
   <222> (1)..(33)
<400> 29
   tggtgatgag gctagctaca acgacatctc tgt 33
<210> 30
   <211> 33
   <212> DNA
   <213> Homo sapiens
<220>
   <221> td30 DNAzyme against T-bet mRNA
   <222> (1)..(33)
<400> 30
   gcttggtgag gctagctaca acgagatcat ctc 33
<210> 31
   <211> 33
   <212> DNA
   <213> Homo sapiens
<220>
   <221> td31 DNAzyme against T-bet mRNA
   <222> (1)..(33)
<400> 31
   atgggaacag gctagctaca acgaccgccg tcc 33
<210> 32
   <211> 33
   <212> DNA
   <213> Homo sapiens
<220>
   <221> td32 DNAzyme against T-bet mRNA
   <222> (1)..(33)
<400> 32
   gaatgggaag gctagctaca acgaatccgc cgt 33
<210> 33
   <211> 33
   <212> DNA
   <213> Homo sapiens
<220>
   <221> td33 DNAzyme against T-bet mRNA
   <222> (1)..(33)
<400> 33
   tgacaggaag gctagctaca acgagggaac atc 33
<210> 34
   <211> 33
   <212> DNA
   <213> Homo sapiens
<220>
   <221> td34 DNAzyme against T-bet mRNA
   <222> (1)..(33)
<400> 34
   agtaaatgag gctagctaca acgaaggaat ggg 33
<210> 35
   <211> 33
   <212> DNA
   <213> Homo sapiens
<220>
   <221> td35 DNAzyme against T-bet mRNA
   <222> (1)..(33)
<400> 35
   cacagtaaag gctagctaca acgagacagg aat 33
<210> 36
   <211> 33
   <212> DNA
   <213> Homo sapiens
<220>
   <221> td36 DNAzyme against T-bet mRNA
   <222> (1)..(33)
<400> 36
   gcccggccag gctagctaca acgaagtaaa tga 33
<210> 37
   <211> 33
   <212> DNA
   <213> Homo sapiens
<220>
   <221> td37 DNAzyme against T-bet mRNA
   <222> (1)..(33)
<400> 37
   ccacaaacag gctagctaca acgacctgta gtg 33
<210> 38
   <211> 33
   <212> DNA
   <213> Homo sapiens
<220>
   <221> td38 DNAzyme against, T-bet mRNA
   <222> (1)..(33)
<400> 38
   gtccacaaag gctagctaca acgaatcctg tag 33
<210> 39
   <211> 33
   <212> DNA
   <213> Homo sapiens
<220>
   <221> td39 DNAzyme against T-bet mRNA
   <222> (1)..(33)
<400> 39
   ccacgtccag gctagctaca acgaaaacat cct 33
<210> 40
   <211> 33
   <212> DNA
   <213> Homo sapiens
<220>
   <221> td40 DNAzyme against T-bet mRNA
   <222> (1)..(33)
<400> 40
   ccaagaccag gctagctaca acgagtccac aaa 33
<210> 41
   <211> 33
   <212> DNA
   <213> Homo sapiens
<220>
   <221> td41 DNAzyme against T-bet mRNA
   <222> (1)..(33)
<400> 41
   ccaccaagag gctagctaca acgacacgtc cac 33
<210> 42
   <211> 33
   <212> DNA
   <213> Homo sapiens
<220>
   <221> td42 DNAzyme against T-bet mRNA
   <222> (1)..(33)
<400> 42
   gctggtccag gctagctaca acgacaagac cac 33
<210> 43
   <211> 33
   <212> DNA
   <213> Homo sapiens
<220>
   <221> td43 DNAzyme against T-bet mRNA
   <222> (1)..(33)
<400> 43
   gctctggtag gctagctaca acgacgccag tgg 33
<210> 44
   <211> 33
   <212> DNA
   <213> Homo sapiens
<220>
   <221> td44 DNAzyme against T-bet mRNA
   <222> (1)..(33)
<400> 44
   ctgcacccag gctagctaca acgattgccg ctc 33
<210> 45
   <211> 33
   <212> DNA
   <213> Homo sapiens
<220>
   <221> td45 DNAzyme against T-bet mRNA
   <222> (1)..(33)
<400> 45
   cacactgcag gctagctaca acgaccactt gcc 33
<210> 46
   <211> 33
   <212> DNA
   <213> Homo sapiens
<220>
   <221> td46 DNAzyme against T-bet mRNA
   <222> (1)..(33)
<400> 46
   ctttccacag gctagctaca acgatgcacc cac 33
<210> 47
   <211> 33
   <212> DNA
   <213> Homo sapiens
<220>
   <221> td47 DNAzyme against T-bet mRNA
   <222> (1)..(33)
<400> 47
   gcctttccag gctagctaca acgaactgca ccc 33
<210> 48
   <211> 33
   <212> DNA
   <213> Homo sapiens
<220>
   <221> td48 DNAzyme against T-bet mRNA
   <222> (1)..(33)
<400> 48
   ttcctggcag gctagctaca acgagctgcc ctc 33
<210> 49
   <211> 33
   <212> DNA
   <213> Homo sapiens
<220>
   <221> td49 DNAzyme against T-bet mRNA
   <222> (1)..(33)
<400> 49
   gtggacgtag gctagctaca acgaaggcgg ttt 33
<210> 50
   <211> 33
   <212> DNA
   <213> Homo sapiens
<220>
   <221> td50 DNAzyme against T-bet mRNA
   <222> (1)..(33)
<400> 50
   ccgggtggag gctagctaca acgagtacag gcg 33
<210> 51
   <211> 33
   <212> DNA
   <213> Homo sapiens
<220>
   <221> td51 DNAzyme against T-bet mRNA
   <222> (1)..(33)
<400> 51
   cctggcgcag gctagctaca acgaccagtg cgc 33
<210> 52
   <211> 33
   <212> DNA
   <213> Homo sapiens
<220>
   <221> td52 DNAzyme against T-bet mRNA
   <222> (1)..(33)
<400> 52
   caaatgaaag gctagctaca acgattcctg gcg 33
<210> 53
   <211> 33
   <212> DNA
   <213> Homo sapiens
<220>
   <221> td53 DNAzyme against T-bet mRNA
   <222> (1)..(33)
<400> 53
   tttcccaaag gctagctaca acgagaaact tcc 33
<210> 54
   <211> 33
   <212> DNA
   <213> Homo sapiens
<220>
   <221> td54 DNAzyme against T-bet mRNA
   <222> (1)..(33)
<400> 54
   attgttggag gctagctaca acgagccccc ttg 33
<210> 55
   <211> 33
   <212> DNA
   <213> Homo sapiens
<220>
   <221> td55 DNAzyme against T-bet mRNA
   <222> (1)..(33)
<400> 55
   tgggtcacag gctagctaca acgatgttgg acg 33
<210> 56
   <211> 33
   <212> DNA
   <213> Homo sapiens
<220>
   <221> td56 DNAzyme against T-bet mRNA
   <222> (1)..(33)
<400> 56
   tctgggtcag gctagctaca acgaattgtt gga 33
<210> 57
   <211> 33
   <212> DNA
   <213> Homo sapiens
<220>
   <221> td57 DNAzyme against T-bet mRNA
   <222> (1)..(33)
<400> 57
   gcacaatcag gctagctaca acgactgggt cac 33
<210> 58
   <211> 33
   <212> DNA
   <213> Homo sapiens
<220>
   <221> td58 DNAzyme against T-bet mRNA
   <222> (1)..(33)
<400> 58
   ggagcacaag gctagctaca acgacatctg ggt 33
<210> 59
   <211> 33
   <212> DNA
   <213> Homo sapiens
<220>
   <221> td59 DNAzyme against T-bet mRNA
   <222> (1)..(33)
<400> 59
   actggagcag gctagctaca acgaaatcat ctg 33
<210> 60
   <211> 33
   <212> DNA
   <213> Homo sapiens
<220>
   <221> td60 DNAzyme against T-bet mRNA
   <222> (1)..(33)
<400> 60
   atggagggag gctagctaca acgatggagc aca 33
<210> 61
   <211> 33
   <212> DNA
   <213> Homo sapiens
<220>
   <221> td61 DNAzyme against T-bet mRNA
   <222> (1)..(33)
<400> 61
   tggtacttag gctagctaca acgaggaggg act 33
<210> 62
   <211> 33
   <212> DNA
   <213> Homo sapiens
<220>
   <221> td62 DNAzyme against T-bet mRNA
   <222> (1)..(33)
<400> 62
   gggctggtag gctagctaca acgattatgg agg 33
<210> 63
   <211> 33
   <212> DNA
   <213> Homo sapiens
<220>
   <221> td63 DNAzyme against T-bet mRNA
   <222> (1)..(33)
<400> 63
   tcaacgatag gctagctaca acgagcagcc ggg 33
<210> 64
   <211> 33
   <212> DNA
   <213> Homo sapiens
<220>
   <221> td64 DNAzyme against T-bet mRNA
   <222> (1)..(33)
<400> 64
   cctcaacgag gctagctaca acgaatgcag ccg 33
<210> 65
   <211> 33
   <212> DNA
   <213> Homo sapiens
<220>
   <221> td65 DNAzyme against T-bet mRNA
   <222> (1)..(33)
<400> 65
   tcacctcaag gctagctaca acgagatatg cag 33
<210> 66
   <211> 33
   <212> DNA
   <213> Homo sapiens
<220>
   <221> td66 DNAzyme against T-bet mRNA
   <222> (1)..(33)
<400> 66
   cgtcgttcag gctagctaca acgactcaac gat 33
<210> 67
   <211> 33
   <212> DNA
   <213> Homo sapiens
<220>
   <221> td67 DNAzyme against T-bet mRNA
   <222> (1)..(33)
<400> 67
   gtaaagatag gctagctaca acgagcgtgt tgg 33
<210> 68
   <211> 33
   <212> DNA
   <213> Homo sapiens
<220>
   <221> td68 DNAzyme against T-bet mRNA
   <222> (1)..(33)
<400> 68
   aagtaaagag gctagctaca acgaatgcgt gtt 33
<210> 69
   <211> 33
   <212> DNA
   <213> Homo sapiens
<220>
   <221> td69 DNAzyme against T-bet mRNA
   <222> (1)..(33)
<400> 69
   ggcaatgaag gctagctaca acgatgggtt tct 33
<210> 70
   <211> 33
   <212> DNA
   <213> Homo sapiens
<220>
   <221> td70 DNAzyme against T-bet mRNA
   <222> (1)..(33)
<400> 70
   tcacggcaag gctagctaca acgagaactg ggt 33
<210> 71
   <211> 33
   <212> DNA
   <213> Homo sapiens
<220>
   <221> td71 DNAzyme against T-bet mRNA
   <222> (1)..(33)
<400> 71
   aggcagtcag gctagctaca acgaggcaat gaa 33
<210> 72
   <211> 33
   <212> DNA
   <213> Homo sapiens
<220>
   <221> td72 DNAzyme against T-bet mRNA
   <222> (1)..(33)
<400> 72
   atctcggcag gctagctaca acgatctggt agg 33
<210> 73
   <211> 33
   <212> DNA
   <213> Homo sapiens
<220>
   <221> td73 DNAzyme against T-bet mRNA
   <222> (1)..(33)
<400> 73
   gctgagtaag gctagctaca acgactcggc att 33
<210> 74
   <211> 33
   <212> DNA
   <213> Homo sapiens
<220>
   <221> td74 DNAzyme against T-bet mRNA
   <222> (1)..(33)
<400> 74
   tattatcaag gctagctaca acgatttcag ctg 33
<210> 75
   <211> 33
   <212> DNA
   <213> Homo sapiens
<220>
   <221> td75 DNAzyme against T-bet mRNA
   <222> (1)..(33)
<400> 75
   gggttattag gctagctaca acgacaattt tca 33
<210> 76
   <211> 33
   <212> DNA
   <213> Homo sapiens
<220>
   <221> td76 DNAzyme against T-bet mRNA
   <222> (1)..(33)
<400> 76
   aaggggttag gctagctaca acgatatcaa ttt 33
<210> 77
   <211> 33
   <212> DNA
   <213> Homo sapiens
<220>
   <221> td77 DNAzyme against T-bet mRNA
   <222> (1)..(33)
<400> 77
   ctcccggaag gctagctaca acgacctttg gca 33
<210> 78
   <211> 33
   <212> DNA
   <213> Homo sapiens
<220>
   <221> td78 DNAzyme against T-bet mRNA
   <222> (1)..(33)
<400> 78
   gtacatggag gctagctaca acgatcaaag ttc 33
<210> 79
   <211> 2588
   <212> DNA
   <213> Homo sapiens
<220>
   <221> td54 bindingsite
   <222> (952)..(970)
<220>
   <221> td69 bindingsite
   <222> (1096)..(1114)
<220>
   <221> td70 bindingsite
   <222> (1100)..(1118)
<400> 79
   cggcccgctg gagaggaagc ccgagagctg ccgcgcgcct gccggacgag ggcgtagaag 60
   ccaggcgtca gagcccgggc tccggtgggg tcccccaccc ggccctcggg tcccccgccc 120
   cctgctccct gcccatccca gcccacgcga ccctctcgcg cgcggagggg cgggtcctcg 180
   acggctacgg gaaggtgcca gcccgccccg gatgggcatc gtggagccgg gttgcggaga 240
   catgctgacg ggcaccgagc cgatgccggg gagcgacgag ggccgggcgc ctggcgccga 300
   cccgcagcac cgctacttct acccggagcc gggcgcgcag gacgcggacg agcgtcgcgg 360
   gggcggcagc ctggggtctc cctacccggg gggcgccttg gtgcccgccc cgccgagccg 420
   cttccttgga gcctacgcct acccgccgcg accccaggcg gccggcttcc ccggcgcggg 480
   cgagtccttc ccgccgcccg cggacgccga gggctaccag ccgggcgagg gctacgccgc 540
   cccggacccg cgcgccgggc tctacccggg gccgcgtgag gactacgcgc tacccgcggg 600
   actggaggtg tcggggaaac tgagggtcgc gctcaacaac cacctgttgt ggtccaagtt 660
   taatcagcac cagacagaga tgatcatcac caagcaggga cggcggatgt tcccattcct 720
   gtcatttact gtggccgggc tggagcccac cagccactac aggatgtttg tggacgtggt 780
   cttggtggac cagcaccact ggcggtacca gagcggcaag tgggtgcagt gtggaaaggc 840
   cgagggcagc atgccaggaa accgcctgta cgtccacccg gactccccca acacaggagc 900
   gcactggatg cgccaggaag tttcatttgg gaaactaaag ctcacaaaca acaagggggc 960
   gtccaacaat gtgacccaga tgattgtgct ccagtccctc cataagtacc agccccggct 1020
   gcatatcgtt gaggtgaacg acggagagcc agaggcagcc tgcaacgctt ccaacacgca 1080
   tatctttact ttccaagaaa cccagttcat tgccgtgact gcctaccaga atgccgagat 1140
   tactcagctg aaaattgata ataacccctt tgccaaagga ttccgggaga actttgagtc 1200
   catgtacaca tctgttgaca ccagcatccc ctccccgcct ggacccaact gtcaattcct 1260
   tgggggagat cactactctc ctctcctacc caaccagtat cctgttccca gccgcttcta 1320
   ccccgacctt cctggccagg cgaaggatgt ggttccccag gcttactggc tgggggcccc 1380
   ccgggaccac agctatgagg ctgagtttcg agcagtcagc atgaagcctg cattcttgcc 1440
   ctctgcccct gggcccacca tgtcctacta ccgaggccag gaggtcctgg cacctggagc 1500
   tggctggcct gtggcacccc agtaccctcc caagatgggc ccggccagct ggttccgccc 1560
   tatgcggact ctgcccatgg aacccggccc tggaggctca gagggacggg gaccagagga 1620
   ccagggtccc cccttggtgt ggactgagat tgcccccatc cggccggaat ccagtgattc 1680
   aggactgggc gaaggagact ctaagaggag gcgcgtgtcc ccctatcctt ccagtggtga 1740
   cagctcctcc cctgctgggg ccccttctcc ttttgataag gaagctgaag gacagtttta 1800
   taactatttt cccaactgag cagatgacat gatgaaagga acagaaacag tgttattagg 1860
   ttggaggaca ccgactaatt tgggaaacgg atgaaggact gagaaggccc ccgctccctc 1920
   tggcccttct ctgtttagta gttggttggg gaagtggggc tcaagaagga ttttggggtt 1980
   caccagatgc ttcctggccc acgatgaaac ctgagagggg tgtccccttg ccccatcctc 2040
   tgccctaact acagtcgttt acctggtgct gcgtcttgct tttggtttcc agctggagaa 2100
   aagaagacaa gaaagtcttg ggcatgaagg agctttttgc atctagtggg tgggaggggt 2160
   caggtgtggg acatgggagc aggagactcc actttcttcc tttgtacagt aactttcaac 2220
   cttttcgttg gcatgtgtgt taatccctga tccaaaaaga acaaatacac gtatgttata 2280
   accatcagcc cgccagggtc agggaaagga ctcacctgac tttggacagc tggcctgggc 2340
   tccccctgct caaacacagt ggggatcaga gaaaaggggc tggaaagggg ggaatggccc 2400
   acatctcaag aagcaagata ttgtttgtgg tggttgtgtg tgggtgtgtg ttttttcttt 2460
   ttctttcttt ttattttttt tgaatggggg aggctattta ttgtactgag agtggtgtct 2520
   ggatatattc cttttgtctt catcactttc tgaaataaac ataaaactgt taaaaaaaaa 2580
   aaaaaaaa 2588
<210> 80
   <211> 2450
   <212> DNA
   <213> Homo sapiens
<220>
   <221> mutation
   <222> (134)..(134)
<220>
   <221> mutation
   <222> (310)..(310)
<220>
   <221> td54 bindingsite
   <222> (952)..(970)
<220>
   <221> td69 bindingsite
   <222> (1096)..(1114)
<220>
   <221> td70 bindingsite
   <222> (1100)..(1118)
<220>
   <221> mutation
   <222> (1399)..(1399)
<220>
   <221> mutation
   <222> (1556)..(1556)
<400> 80
   cggcccgctg gagaggaagc ccgagagctg ccgcgcgcct gccggacgag ggcgtagaag      60
   ccaggcgtca gagcccgggc tccggtgggg tcccccaccc ggccctcggg tcccccgccc     120
   cctgctccct gcctatccca gcccacgcga ccctctcgcg cgcggagggg cgggtcctcg     180
   acggctacgg gaaggtgcca gcccgccccg gatgggcatc gtggagccgg gttgcggaga     240
   catgctgacg ggcaccgagc cgatgccggg gagcgacgag ggccgggcgc ctggcgccga     300
   cccgcagcag cgctacttct acccggagcc gggcgcgcag gacgcggacg agcgtcgcgg     360
   gggcggcagc ctggggtctc cctacccggg gggcgccttg gtgcccgccc cgccgagccg     420
   cttccttgga gcctacgcct acccgccgcg accccaggcg gccggcttcc ccggcgcggg     480
   cgagtccttc ccgccgcccg cggacgccga gggctaccag ccgggcgagg gctacgccgc     540
   cccggacccg cgcgccgggc tctacccggg gccgcgtgag gactacgcgc tacccgcggg     600
   actggaggtg tcggggaaac tgagggtcgc gctcaacaac cacctgttgt ggtccaagtt     660
   taatcagcac cagacagaga tgatcatcac caagcaggga cggcggatgt tcccattcct     720
   gtcatttact gtggccgggc tggagcccac cagccactac aggatgtttg tggacgtggt     780
   cttggtggac cagcaccact ggcggtacca gagcggcaag tgggtgcagt gtggaaaggc     840
   cgagggcagc atgccaggaa accgcctgta cgtccacccg gactccccca acacaggagc     900
   gcactggatg cgccaggaag tttcatttgg gaaactaaag ctcacaaaca acaagggggc     960
   gtccaacaat gtgacccaga tgattgtgct ccagtccctc cataagtacc agccccggct    1020
   gcatatcgtt gaggtgaacg acggagagcc agaggcagcc tgcaacgctt ccaacacgca    1080
   tatctttact ttccaagaaa cccagttcat tgccgtgact gcctaccaga atgccgagat    1140
   tactcagctg aaaattgata ataacccctt tgccaaagga ttccgggaga actttgagtc   1200
   catgtacaca tctgttgaca ccagcatccc ctccccgcct ggacccaact gtcaattcct   1260
   tgggggagat cactactctc ctctcctacc caaccagtat cctgttccca gccgcttcta   1320
   ccccgacctt cctggccagg cgaaggatgt ggttccccag gcttactggc tgggggcccc   1380
   ccgggaccac agctatgggg ctgagtttcg agcagtcagc atgaagcctg cattcttgcc   1440
   ctctgcccct gggcccacca tgtcctacta ccgaggccag gaggtcctgg cacctggagc   1500
   tggctggcct gtggcacccc agtaccctcc caagatgggc ccggccagct ggttcagccc   1560
   tatgcggact ctgcccatgg aacccggccc tggaggctca gagggacggg gaccagagga   1620
   ccagggtccc cccttggtgt ggactgagat tgcccccatc cggccggaat ccagtgattc   1680
   aggactgggc gaaggagact ctaagaggag gcgcgtgtcc ccctatcctt ccagtggtga   1740
   cagctcctcc cctgctgggg ccccttctcc ttttgataag gaagctgaag gacagtttta   1800
   taactatttt cccaactgag cagatgacat gatgaaagga acagaaacag tgttattagg   1860
   ttggaggaca ccgactaatt tgggaaacgg atgaaggact gagaaggccc ccgctccctc   1920
   tggcccttct ctgtttagta gttggttggg gaagtggggc tcaagaagga ttttggggtt   1980
   caccagatgc ttcctggccc acgatgaaac ctgagagggg tgtccccttg ccccatcctc   2040
   tgccctaact acagtcgttt acctggtgct gcgtcttgct tttggtttcc agctggagaa   2100
   aagaagacaa gaaagtcttg ggcatgaagg agctttttgc atctagtggg tgggaggggt   2160
   caggtgtggg acatgggagc aggagactcc actttcttcc tttgtacagt aactttcaac   2220
   cttttcgttg gcatgtgtgt taatccctga tccaaaaaga acaaatacac gtatgttata   2280
   accatcagcc cgccagggtc agggaaagga ctcacctgac tttggacagc tggcctgggc   2340
   tccccctgct caaacacagt ggggatcaga gaaaaggggc tggaaagggg ggaatggccc   2400
   acatctcaag aagcaagata ttgtttgtgg tggttgtgtg tgggtgtgtg              2450

## Beschreibung

Die vorliegende Erfindung betrifft ein Verfahren zur Herstellung eines Zell und/oder Gewebe- und/oder Krankheitsphasen-spezifischen Arzneimittels, das zur Behandlung von chronischen Entzündungen geeignet ist.

### Hintergrund der Erfindung

Chronische Entzündungen stellen einen zunehmend großen medizinischen Problemkreis mit hohem sozioökonomischen Impakt dar. Hierzu zählen insbesondere folgende Erkrankungsgruppen:
- Autoimmunerkrankungen und Erkrankungen des rheumatischen Formenkreises (Manifestationen an u.a. Haut, Lunge, Niere, Gefäßsystem, Nervensystem, Bindegewebe, Bewegungsapparat, endokrinem System)
- Allergische Soforttypreaktionen und Asthma
- Chronisch-obstruktive Lungenerkrankungen (COPD)
- Arteriosklerose
- Psoriasis und Kontaktekzem
- Chronische Abstoßungsreaktionen nach Organ-, Knochenmarkstransplantation

Viele dieser Erkrankungen zeigen in den letzten Dekaden eine ansteigende Prävalenz nicht nur in den Industrienationen, sondern zum Teil weltweit. So leiden in Europa, Nordamerika, Japan und Australien mittlerweile über 20% der Bevölkerung an allergischen Erkrankungen und Asthma. Chronisch-obstruktive Lungenerkrankungen sind zurzeit die fünfthäufigste Todesursache weltweit und werden nach Berechnungen der WHO im Jahre 2020 die dritthäufigste Todesursache darstellen. Arteriosklerose mit den Folgeerkrankungen Herzinfarkt, Schlaganfall und peripherer arterielle Verschlusskrankheit nehmen in der Morbiditäts- und Mortalitätsstatistik weltweit eine führende Position ein. Psoriasis und Kontaktekzem sind zusammen mit der Neurodermitis die häufigsten chronischen Entzündungserkrankungen an der Haut überhaupt.

Aufgrund von bis heute nur unzureichend verstandenen Wechselwirkungen zwischen Umweltfaktoren und einer genetischen Disposition kommt es zu nachhaltigen Fehlregulationen des Immunsystems. Hierbei lassen sich für diese unterschiedlichen Erkrankungen folgende gemeinsame Prinzipien feststellen:
(A) Es kommt zur Entwicklung einer überschießenden Immunantwort gegen normalerweise für den Menschen harmlose Antigene. Diese Antigene können Bestandteile der Umwelt sein (z. B. Allergene, wie Pollen, Tierhaare, Nahrungsmittel, Milben, chemische Substanzen wie Konservierungsstoffe, Farbstoffe, Reinigungsmittel). In diesen Fällen entwickelt sich bei den Patienten eine allergische Reaktion. Im Falle von z.B. Aktiv- und Passiv-Zigarettenrauchern kommt es zu chronisch-obstruktiven Lungenerkrankungen (COPD). Andererseits kann das Immunsystem aber auch gegen Komponenten des eigenen Organismus reagieren, diese als fremd erkennen und eine Entzündungsreaktion dagegen in Gang setzen. In diesen Fällen entwickelt sich eine Autoimmunerkrankung. In jedem Falle werden harmlose, nicht-toxische Antigene fälschlicherweise als fremd bzw. gefährlich erkannt und eine unangemessene Entzündungsreaktion in Gang gesetzt.
(B) Die Erkrankungen verlaufen in Phasen zu denen die Initiation, Progression, also Fortschreiten der Entzündungsreaktion, und die damit assoziierte Destruktion und der Umbau mit Verlust von Organ-Funktionalität (sogenanntes Remodeling) zählen.
(C) Die Erkrankungen zeigen Patienten-spezifische sub-phänotypische Ausprägungsmerkmale.
(D)An der Initiation, Aufrechterhaltung und den Destruktions- und Umbauprozessen sind Komponenten der angeborenen und erworbenen Immunität nachhaltig beteiligt. Unter dem Einfluss der angeborenen Immunität (wichtige Komponenten: Antigen-präsentierende-Zellen mit ihren diversen Populationen und das Komplementsystem) kommt es zu Aktivierung und Differenzierung der Zellen des adaptiven Immunsystems (wichtige Komponenten: T- und B-Lymphozyten). Die T-Zellen übernehmen zentrale Funktionen im weiteren Verlauf indem sie in hoch-spezialisierte Effektoren differenzieren. Hierbei aktivieren und erwerben sie bestimmte Effektormechanismen, zu denen insbesondere folgende Funktionen zählen: Antikörperproduktion, Kontrolle der Funktionalität von Effektorzellen des Immunsystems (wie z. B. neutrophile-, basophile-, eosinophile Granulozyten), Rückkopplung auf Funktionen des angeborenen Immunsystems, Beeinflussung der Funktionalität von nichthämatopoetischen Zellen wie z. B. Epithel, Endothel, Bindegewebe, Knochen und Knorpel und vor allem neuronale Zellen. Hier kommt es zu einer besonderen Wechselwirkung zwischen Immun- und Nervensystem, aus dem sich das Konzept der Neuro-immunologischen Interaktion bei chronischen Entzündungen entwickelt hat

Aufgrund der Komplexität und Vielschichtigkeit der Krankheitsbilder, die mit chronischen Entzündungen einhergehen, müssen an ein optimales Arzneimittel zur Behandlung der Krankheiten folgende Anforderungen gestellt werden:
(1) Erkrankungen manifestieren sich in Patienten-spezifischen (Sub)-Phänotypen. Arzneimittel müssen daher eine hohe Patienten- bzw. Fallspezifität aufweisen.
(2) Erkrankungen verlaufen in Stadien und Phasen. Arzneimittel müssen daher eine Stadien- bzw. Phasenspezifität besitzen.
(3) Die Erkrankungen werden von unterschiedlich spezialisierten Zellen reguliert. Die Arzneimittel müssen daher eine Zell-spezifische Intervention bewirken.
(4) Die Erkrankungen manifestieren sich an unterschiedlichen Organen und Kompartimenten. Die Arzneimittel müssen daher eine Kompartiment- bzw. Organ-Spezifität besitzen.
(5) Arzneimittel müssen für eine Langzeittherapie geeignet sein. So müssen Reaktionen des Immunsystems gegen die Arzneimittel verhindert werden.
(6) Das Nebenwirkungsprofil der Arzneimittel muss in medizinischer und ethischer Relation zu Schweregrad, Prognose und Verlauf der Erkrankungen in einem akzeptablen Verhältnis stehen.

Keine der heute verfügbaren, etablierten Therapien gegen chronische Entzündungen erfüllt diese Kriterien optimal. Bekannt sind aus der DE 695 11 245 T2 die Behandlung mit Immunglobulin A und aus der DE 695 18 667 T2 die Hemmung der Phospholipase A₂ (PLA₂) und /oder Coenzym-A-unabhängigen Transacylase (CoA-IT). Im Mittelpunkt der heute etablierten Therapiekonzepte stehen für diese Erkrankung die unspezifische anti-inflammatorische Therapie, sowie die Immunsuppression. So sind viele der eingesetzten unspezifischen anti-inflammatorisch wirkenden Substanzen wie Ibuprofen, Acetylsalicylsäure und Paracetamol entweder nicht wirksam genug oder mit einer hohen Rate unerwünschter Nebenwirkungen behaftet. Steroide haben dagegen zwar eine höhere Wirkungspotenz, sind aber ihrerseits mit schwerwiegenden Nebenwirkungen wie Hypertonus, Diabetes und Osteoporose behaftet. Immunsuppressive Medikamente der neueren Generation wie z. B. Cyclosporin und Tacrolimus zeigen Hepato- und Nephrotoxizität.

Diese Situation hat zur Suche und klinischen Erprobung einer Vielzahl von neueren Molekülen geführt, die spezifischer in die immunologischen und zellbiologischen Fehlregulationen eingreifen sollen. Hierzu zählen Zytokine, Zytokin-Rezeptoren und Anti-Zytokine. Probleme, die mit diesen neueren therapeutischen Einsätzen verbunden sind, schließen mangelnde Zell- und Organ-Spezifität. Entwicklung von unerwünschten Immunreaktionen gegen diese Moleküle, sowie eine mangelnde Wirksamkeit bei verschiedenen Phänotypen ein.

Es wird in neuerer Zeit versucht, eine neue Klasse katalytischer Moleküle, die so genannten "DNAzyme" (Santoro, 1997) als therapeutische Agenzien zur Inaktivierung von Genen einzusetzen, deren Expression Krankheiten verursacht. DNAzyme sind Einzelstrang-Moleküle, die prinzipiell an komplementäre Bereiche der RNA binden können und diese durch Spaltung inaktivieren. Der spezifische Einsatz von DNAzymen als therapeutische Agenzien setzt allerdings voraus, dass die krankheitsverursachenden Gene und deren mRNA genauestens bekannt sind. Dies ist bislang nur bei wenigen Erkrankungen der Fall.

Das in der WO 01/11023A1 beschriebene DNAzym bindet RelA (p65) mONA und ist damit gegen den Transkriptionsfaktor NF-κB gerichtet, in der WO 00/42173 ist ein EGR-1 mRNA bindendes DNAzym offenbart. Die W099/50452 offenbart ein 10-23 DNAzym, das in einem diagnostischen Verfahren zum Auffinden von Nukleinsäure-Mutationen verwendet werden kann.
Keine der derzeit bekannten Antisense-Moleküle und DNAzyme können zur Herstellung eines Arzneimittels zur Behandlung von chronischen Entzündungen in Patienten verwendet werden.

### Aufgabe der Erfindung

Aufgabe der vorliegenden Erfindung ist es, Zell- und/oder Gewebe- und/oder Krankheitsphasen-spezifische Arzneimittel bereitzustellen, die zur funktionellen Inaktivierung von Ribonukleinsäure-Molekülen von Transkriptionsfaktoren und Faktoren der Signaltransduktionswege, deren Expression an der Entstehung von chronischen Entzündungsreaktionen und Autoimmunerkrankungen beteiligt ist, führen und die zur Behandlung von chronischen Entzündungsreaktionen und Autoimmunerkrankungen geeignet sind, wobei die geschilderten Nachteile im Stand der Technik beseitigt werden.
Darüber hinaus ist es Aufgabe der Erfindung, ein Verfahren zur Herstellung Zell und/oder Gewebe- und/oder Krankheitsphasen-spezifischer Arzneimittel bereitzustellen, das Ribonukleinsäure-Moleküle von Transkriptionsfaktoren und von Faktoren der Signaltransduktionswege, deren Expression an der Entstehung von chronischen Entzündungsreaktionen und Autoimmunerkrankungen beteiligt ist, identifiziert und sie in Zielzellen funktionell inaktiviert.

Die Aufgabe wird erfindungsgemäß durch spezifische DNAzyme gemäß den Ansprüchen 1 bis 5, ein Verfahren gemäß Anspruch 6 und ein Arzneimittel sowie dessen Verwendung gemäß den Ansprüchen 7 bis 9 gelöst.
Der Vorteil der Erfindung besteht in einer funktionellen Inaktivierung von Ribonukleinsäure-Molekülen von Transkriptionsfaktoren und Faktoren der Signaltransduktionswege zur Differenzierung und/oder Expression von Zytokinen, die an der Entstehung der chronischen Entzündungsreaktionen und Autoimmunerkrankungen beteiligt sind, mittels spezifischer DNAzyme und/oder siRNA. Diese Strategie zeichnet sich gegenüber konventionellen, aber auch gentherapeutischen Ansätzen durch höchste Zell- und/oder Gewebe- und/oder Krankheitsphasen-Spezifität und -Selektivität, hohe Stabilität der Moleküle und eine vernachlässigbare Antigenität aus. Es werden optimale Voraussetzungen für eine maßgeschneiderte Langzeittherapie bei Patienten mit chronischen Entzündungserkrankungen geschaffen.

Weitere Details und Vorzüge der vorliegenden Erfindung werden aus der folgenden Figur und der Beschreibung ersichtlich. Dabei zeigt
- **Fig. 1:**: schematische Darstellung der Signaltransduktion bei der Differenzierung von CD4⁺ Zellen zu TH1- bzw. TH2-Zell (modifiziert nach Ho I.C. und Glimcher L.H., Cell 2002; 109: S109-S120).
- **Fig. 2:**: Nukleotidsequenz der katalytischen Domäne des 10-23 DNAzym und Bindung an eine Ziel RNA mittels Watson-Crick Paarung. (R = A oder G; Y = U oder C, N = A, G, U oder G). Der Pfeil zeigt die Spaltstelle in der Ziel mRNA.
- **Fig. 3:**: Pool an spezifischen Ribonukleinsäure Molekülen nach Schritt b) im besonderen die DNAzyme td 1 bis td 70 gegen T-bet und ihre Nukleotidsequenzen (A=Adenin, G=Guanin, C=Cytosin, T=Thymin)
- **Fig. 4:**: Nukleotidsequenzen humaner T-bet-Gene im Alignment
Sequenz 1: Humanes T-bet aus Datenbank Nr.: NM_013351.
Sequenz 2: Humanes T-bet (sequenziert aus pBluescript-SK).
Divergente Basen sind grau unterlegt, Primerlokalisationen für die Klonierung von T-bet sind unterstrichen. Die Primerlokalisationen für die relative Quantifizierung im LightCycler sind umrandet. Die Lokalisation der DNAzyme td54 und td69 ist gleichzeitig grau unterlegt und unterstrichen, td70 ist zudem in fettgeschriebenen Buchstaben hervorgehoben.
(A=Adenin, G=Guanin, C=Cytosin, T=Thymin)

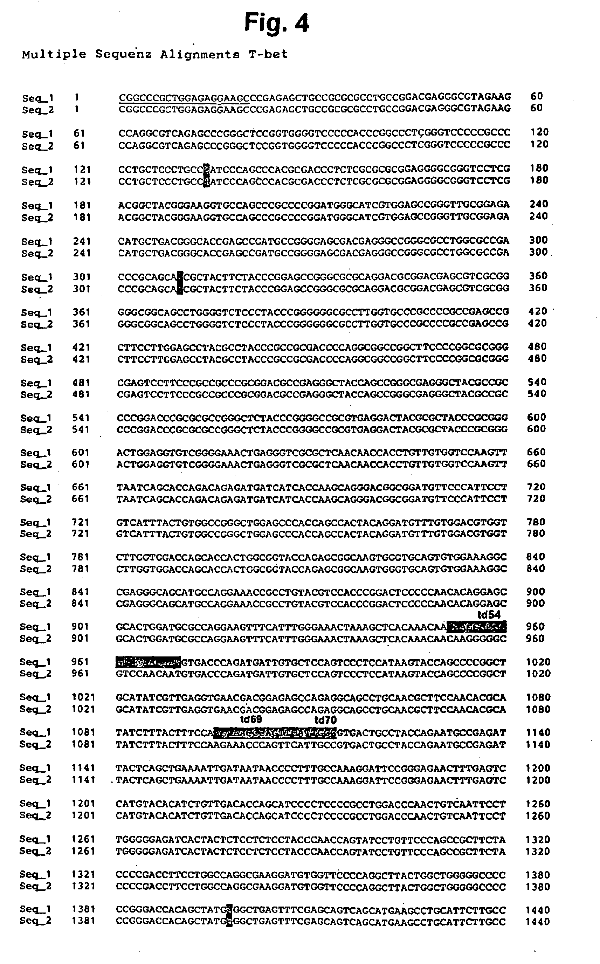

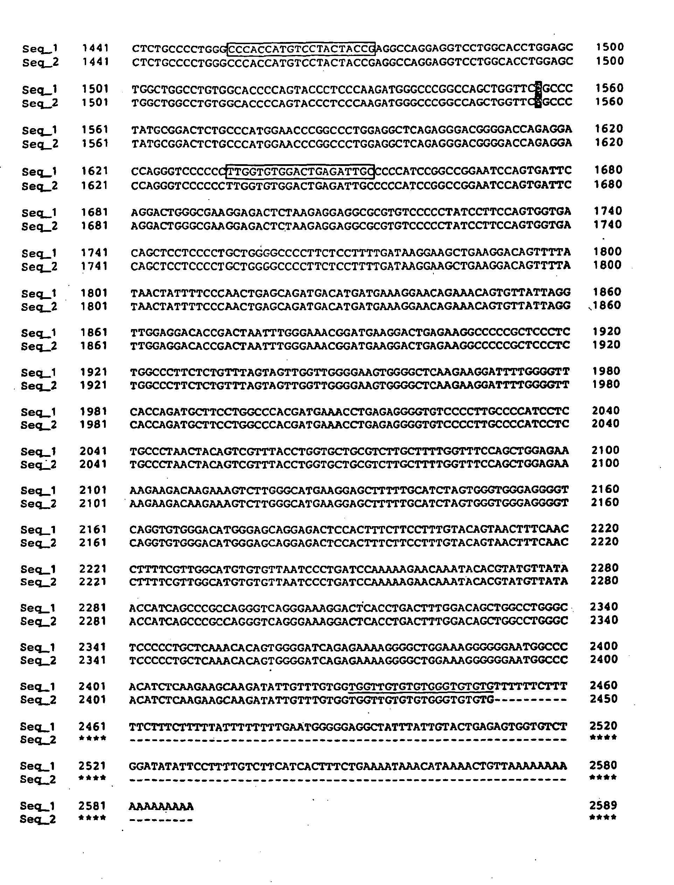

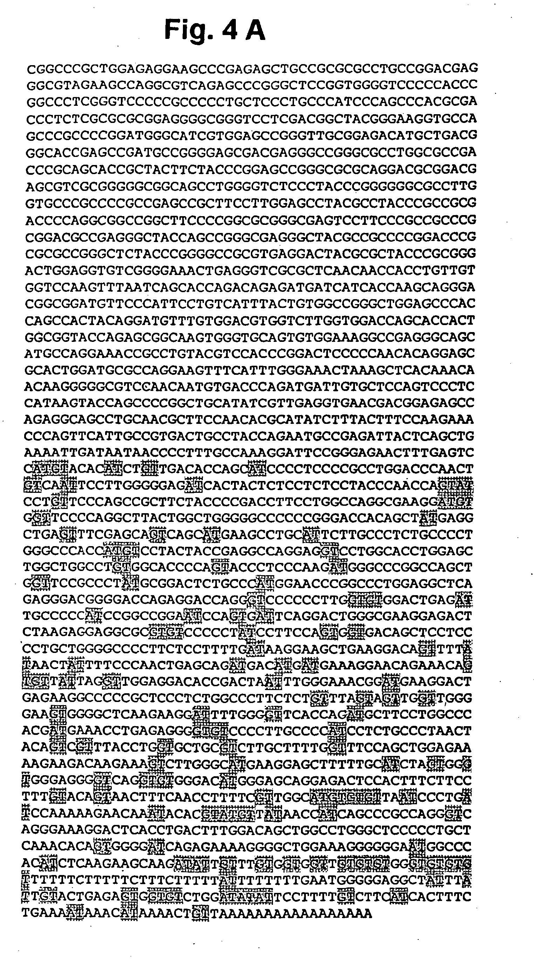

- **Fig. 4A:**: Nukleotidsequenz 1 des humanen T-bet-Gen aus Figur 4, darin als graue Hinterlegung eingezeichnet jeweils die Nukleotidpaare GT und AT, zwischen denen weitere DNAzyme-Schnittstellen liegen.

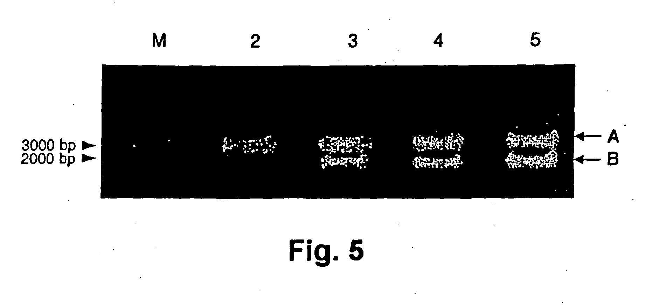

- **Fig. 5:**: Gelelektrophorese zeigt die Spaltung einer Ziel-mRNA (hier T-bet mRNA) mit spezifischen Ribonukleinsäure Molekülen nach Schritt b), hier modifizierte DNAzyme [(td54m (Spur 3), td69m (Spur 4) und td70rn (Spur 5)], Die modifizierten DNAzyme (0,25 µM) werden 30 min bei 37°C mit in vitro transkribierter T-bet mRNA (0,025 µM) in einem Volumen von 10 µl mit folgender Reaktionszusammensetzung inkubiert: 50 mM Tris pH 7,4, 150 mM NaCl, 10 mM MgCl2. Anschließend werden die Produkte gelelektrophoretisch aufgetrennt. Spur M enthält mitgeführten Längenstandard 3000 Basen und 2000 Basen, Spur 2 enthält als Kontrolle mRNA ohne DNAzym-Zugabe. Pfeil A zeigt auf die Bande mit Substrat (hier T-bet-mRNA), Pfeil B auf das größere Spaltprodukt. Das zweite Spaltprodukt ist kleiner und in dieser Abbildung nicht mehr

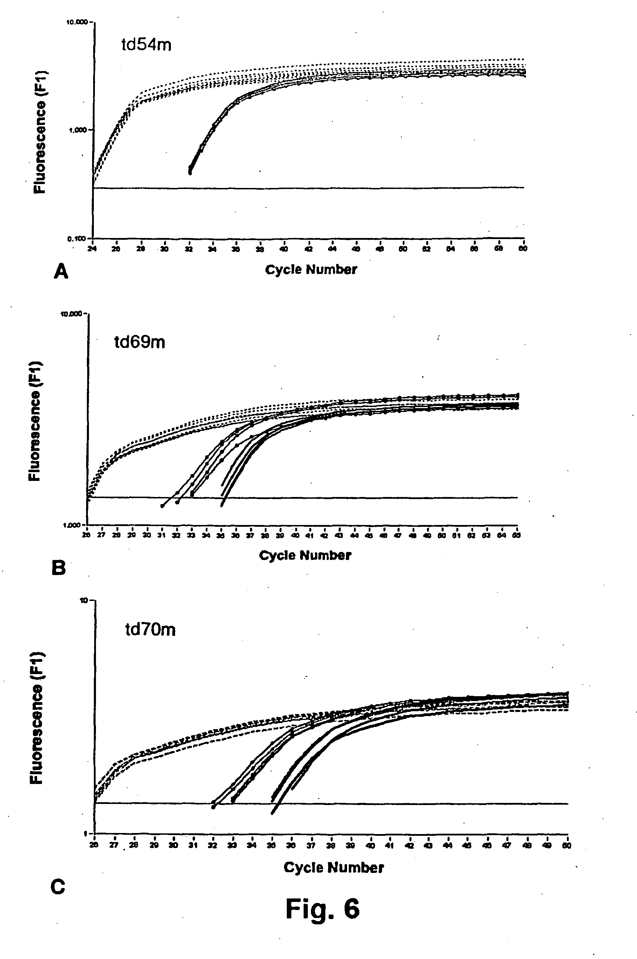

- **Fig. 6:**: Qualifizierung von T-bet- und GAPDH-mRNA Mengen im LightCycler aus, mit DNAzymen td54 (A), td69 (B) und td70 (C) behandelten Zellen. Jurkat E6.1 Zellen werden zweimal in 24h Abstand entweder mit den T-betspezifischen DNAzymen td54 (A), td69 (B) und td70 (C) oder mit Nonsense-DNAzym als Kontrolle (nicht dargestellt) transfiziert. Anschließend wird RNA gereinigt, eine reverse Transkription durchgeführt und die gewonnene DNA im LightCycler eingesetzt. Als interner Standard dient GAPDH (gestrichelte Kurven). Gezeigt sind jeweils 4-fach Bestimmungen von mit T-betspezifischen DNAzymen oder Nonsense-DNAzym behandelten Zellen. Durchgezogene Kurven zeigen die Menge an T-bet in den mit T-betspezifischen DNAzymen behandelten Zellen, gepunkte Linien zeigen die Menge an T-bet in den mit Nonsense-DNAzyme behandelten Zellen.

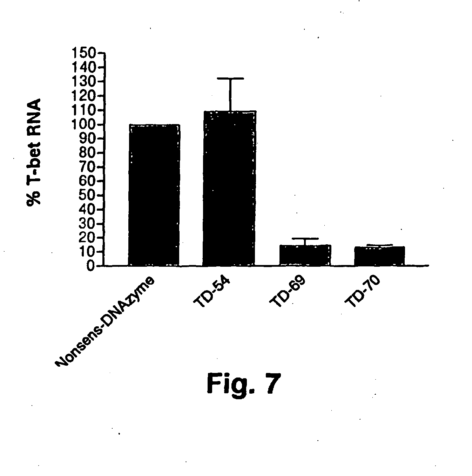

- **Fig.7:**: Diagramm der relativen Quantifizierung von T-bet-mRNA in Jurkat E6.1 Zellen.
Jurkat E6.1 Zellen werden mit den T-bet-spezifischen DNAzymen td54, td69 und td70 zwei mal transfiziert und nach 48h wird RNA isoliert. Nach einer reversen Transkription wird die mRNA-Menge mittels LightCycler bestimmt. Als Kontrolle dient Nonsense-DNAzym. Die relative Quantifizierung von T-bet- und GAPDH-mRNA erfolgt nach Anleitung [beschrieben im User Bulletin #2 (ABI Prism 7700 Sequence detection System User Bulletin #2 (2001). Relative quantification of gene expression.
Http://docs.appliedbiosystems.com/pebiodocs/04303859.pdf)].
Dabei werden die Menge an T-bet-mRNA aus dem Kontrollversuch mit Nonsense-DNAzym gleich 100% gesetzt.

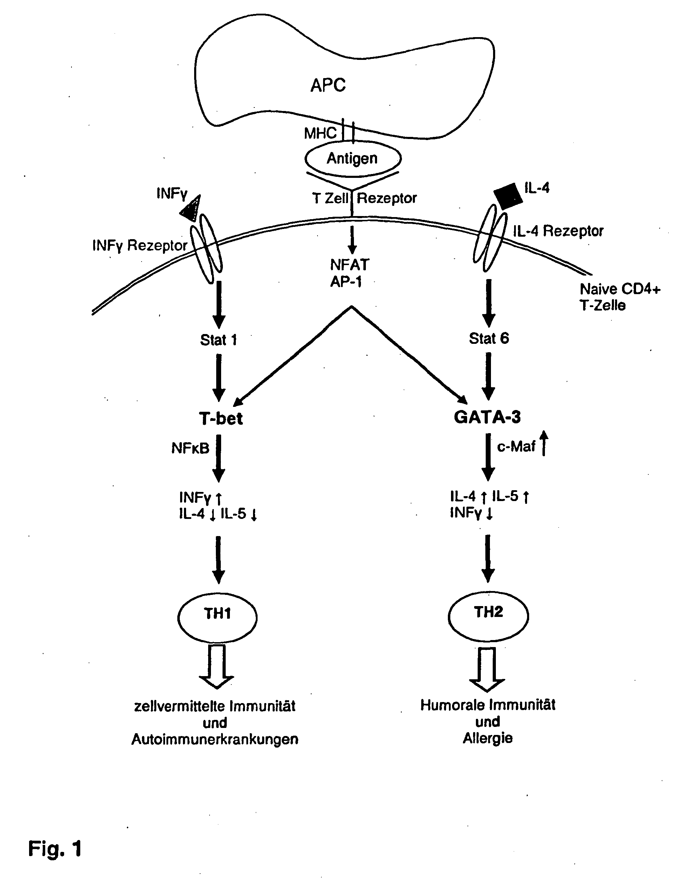

Figur 1 zeigt in einer nach Ho I.C. und Glimcher L.H.-(Cell 2002; 109: S109- S120) modifizierten schematischen Darstellung die Zusammenhänge der Signaltransduktion bei der Differenzierung von CD4⁺ Zellen zu TH1- bzw. TH2-Zell. Die Stimulation über den T-Zellrezeptor durch den entsprechenden Peptid-MHC Komplex induziert die klonale Expansion und programmierte Differenzierung von CD4+ T-Lymphozten zu T-Helfer (TH)1- oder TH2-Zellen. Die Unterscheidung dieser beiden Subtypen erfolgt aufgrund ihrer Zytokin-Profile. TH1-Zellen produzieren Interferon-γ (INFγ), Interleukin 2 (IL-2) und Tumor-Nekrose-Faktor-β, wohingegen TH2-Zellen IL-4, IL-5, IL-9 und IL--13 sezernieren. Bakterielle und virale Infektionen induzieren eine Immunantwort, die von TH1-Zellen dominiert wird. Auf der anderen Seite regulieren TH2-Zellen die IgE Produktion gegen Parasiten. Dabei besteht zwischen TH1- und TH2-Zellen ein Gleichgewicht. Die Zerstörung dieses Gleichgewichtes verursacht Krankheiten, so ist eine überschießende TH1-Zellenantwort assoziiert mit Autoimmunerkrankungen, während allergischen Erkrankungen eine verstärkte TH2-Zellantwort zugrunde liegt.

Es ist bekannt, dass TH1-Zytokine in die Pathogenese von Autoimmunerkrankungen wie z.B. Autoimmunuveitis, experimentelle allergische Enzephalomyelitis, Typ 1 Diabetes mellitus oder Morbus Crohn involviert sind, während TH2-Zytokine (IL A, IL-5, IL-13 bzw. IL-9) an der Entstehung von chronisch entzündlichen Atemwegserkrankungen wie z.B. Atemwegseosinophilie, Mukus-Hypersekretion und Atemwegs-Hyperreagibilität beteiligt sind. Grundlage dieser Erkrankungen sind pathophysiologische Veränderungen während der Produktion von charakteristischen Zytokinen durch antigenspezifische TH-Zellen. So zeigen transgene Mäuse, die in den Atemwegsepithelien die TH2-Zytokine IL-4, IL-5, IL-13 bzw. IL-9 konstitutiv überexprimieren, typische allergische Entzündungsreaktionen. TH2-Zell-Subpopulationen in der Lunge und den Atemwegen rufen in TH2-Zellen im Tiermodell die charakteristischen Symptome des Asthma bronchiale hervor.

Überraschenderweise wurde gefunden, dass zur Zell- und/oder Gewebespezifischen Behandlung von chronischen Entzündungen und/oder Autoimmunerkrankungen Transkriptionsfaktoren und Faktoren der Signaltransduktionswege zur Differenzierung und/oder Expression von Zytokinen, die an der Entstehung der chronischen Entzündungsreaktionen und Autoimmunerkrankungen beteiligt sind wie z.B.: der TH1-Zell-spezifische Transkriptionsfaktor T-bet und der TH2-Zellspezifische Transkriptionsfaktor GATA-3 in idealer Weise geeignet sind.

Der TH1-Zell-spezifische Transkriptionsfaktor T-bet ist vor allem für die Differenzierung von naiven CD⁺ T-Zellen zu TH1-Zellen verantwortlich. Seine Expression wird über die Signaltransduktionswege des T-Zell Rezeptors (TZR) und über INFγ-Rezeptor/STAT1 kontrolliert. T-bet transaktiviert das endogene INFγ-Gen und induziert die INFγ Produktion. Darüber hinaus induziert er die Hochregulation der Protein-Expression von IL-12Rβ2 Ketten und führt zum Chromatin-Remodeling von individuellen INFγ Allelen. Die in vivo Funktion von T-bet wird in Knock-Out-Mäusen (T-bet^{-/-}) bestätigt. Obwohl T-bet defiziente Mäuse eine normale Lymphozyten Entwicklung aufweisen, produzieren CD4⁺ T-Zellen aus diesen Mäusen kein INFγ, weder auf die Stimulation mit anti-CD3/CD28 noch mit PMA/lonomycin. T-bet defiziente Mäuse zeigen keine Immunantwort auf eine L. major Infektion, die Menge an TH2-Zytokinen ist erhöht.
Bekannt ist die Funktion von T-bet in mucosalen T-Zellen bei der Entstehung von entzündlichen Darmerkrankungen. Untersuchungen im Tiermodell zeigen eine Verschlimmerung der Colitis in rekonstituierten SCID (Severe Combined Immunodeficiency) Mäusen nach retroviraler Transduktion von T-bet in CD4⁺CD26L⁺ T-Zellen, umgekehrt führt der Transfer von T-bet defizienten T-Zellen zu keiner Colitis -Induktion.
Der Transkriptionsfaktor T-bet induziert spezifisch die Entwicklung von TH1-Zellen und kontrolliert die INFγ-Produktion in diesen Zellen. Durch die Inhibition von T-bet wird die Balance zwischen TH1- und TH2-Zellen zugunsten von TH2-Zellen verschoben.

Weitere Transkriptionsfaktoren, die eine Rolle bei der Differenzierung zu TH1-beziehungsweise TH2-Zellen spielen und an der Entstehung der chronischen Entzündungsreaktionen und Autoimmunerkrankungen beteiligt sind weisen eine Expression auf, die sich in einer Zielzelle im Vergleich zur Expression einer Kontrollzelle unterscheidet und werden erfindungsgemäß ebenfalls zum Design von spezifischen DNAzymen und/oder siRNA für den therapeutischen Einsatz bei chronisch entzündlichen Erkrankungen eingesetzt:
- STAT4, STAT5a und STAT1 (signal transducer and activator of transcription)
- c-Rel
- CREB2 (cAMP response element-binding protein 2)
- ATF-2, ATF-2
- Hlx
- IRF-1 (interferon regulatory factor-1)
- c-Maf
- NFAT (Nuclear factor of activated T cells)
- NIP45 (NF-AT interacting protein 45)
- AP1 (Activator Protein 1)
- Mel-18
- SKAT-2 (SCAN box, KRAB domain associated with a Th2 phenotype)
- CTLA-4 (Cytolytic T lymphocyte-associated antigen 4)

Weitere Faktoren der Signaltransduktionswege, die zur Differenzierung und/oder Expression von Zytokinen verantwortlich sind und an der Entstehung der chronischen Entzündungsreaktionen und Autoimmunerkrankungen beteiligt sind, weisen eine Expression auf, die sich in einer Zielzelle im Vergleich zur Expression einer Kontrollzelle unterscheidet und werden erfindungsgemäß ebenfalls zum Design von spezifischen DNAzymen und/oder siRNA für den therapeutischen Einsatz bei chronisch entzündlichen Erkrankungen eingesetzt:
- Src kinase
- Tec kinase
   Rlk (Txk im Menschen)
   Itk
   Tec
- RIBP (Rlk/Itk-binding protein)
- PLCγ (phospholipase Cγ1)
- MAP kinase (Mitogen-activated protein kinase)
   ERK
   JNK
   P38
- MKK (MAP kinase kinase)
   MKK1
   MKK2
   MKK3
   MKK4
   MKK6
   MKK7
- Rac2
- GADD45 (Growth arrest and DNA damage gene 45)
   GADD45β
   GADD45γ
- SOCS (Suppressors of cytokine signalling)
   CIS (Cytokine-induced SH2 protein)
   SOCS1
   SOCS2
   SOCS3
- JAK (Janus kinase)
   JAK1
   JAK3
- NIP45 (NF-AT interacting protein)

Erfindungsgemäß wird ein Zell- und/oder Gewebe- und/oder Krankheitsphasen spezifisches Arzneimittel bereitgestellt, das zur Behandlung von chronischen Entzündungen geeignet ist.
Das Arzneimittel greift bevorzugt an den Interventionspunkten der den chronischen Entzündungsreaktionen und Autoimmunerkrankungen zugrunde liegenden komplexen Kaskade der immunologischen und zellbiologischen Fehlregulationen an. Besonders bevorzugt sind dies Interventionspunkte der Regulation der Differenzierung der beteiligten Transkriptionsfaktoren, wie beispielsweise der TH1-Zell-spezifische Transkriptionsfaktor T-bet. Der erzielte therapeutische Effekt besteht in einer funktionellen Inaktivierung von mRNA-Molekülen mittels spezifischer DNAzyme und/oder siRNA. Diese Strategie bietet eine Reihe von Vorteilen gegenüber konventionellen, aber auch gentherapeutischen Ansätzen: höchste Spezifität und Selektivität, hohe Stabilität der Moleküle und eine vernachlässigbare Antigenität. Es werden optimale Voraussetzungen geschaffen für eine maßgeschneiderte Langzeittherapie bei Patienten mit chronischen Entzündungserkrankungen.

Erfindungsgemäß wird ein Verfahren zur Herstellung eines Zell- und/oder Gewebe- und/oder Krankheitsphasen-spezifischen Arzneimittels bereitgestellt, dass folgende Schritte umfasst:
a) Identifikation von Ribonukleinsäure-Molekülen, deren Expression sich in einer Zielzelle im Vergleich zur Expression einer Kontrollzelle unterscheidet
b) Design von spezifischen Ribonukleinsäure-Molekülen, die an Ribonukleinsäure-Moleküle aus Schritt a) binden und sie funktionell inaktivieren
c) Einbringen der spezifischen Ribonukleinsäure-Moleküle aus Schritt b) in Zielzellen
d) Formulierung der spezifischen Ribonukleinsäure-Molekülen aus Schritt b) und/oder einer Zielzelle aus Schritt c) in einem Arzneimittel

Der Begriff "Zell- und/oder Gewebe- und/oder Krankheitsphasen-spezifisch" bedeutet im Sinne der vorliegenden Erfindung, dass das mittels des erfindungsgemäßen Verfahrens hergestellte Arzneimittel im wesentlichen nur bei einer bestimmten Art von Zellen (Zielzelle) und/oder in bestimmten Geweben oder Organen und/oder in bestimmten Phasen der Erkrankung wirksam ist, und einen vernachlässigbaren Einfluss auf andere Zellen (Kontrollzellen), Gewebe oder Organe besitzt. Vorzugsweise ist das Arzneimittel bei mindestens 2/3 der Zielzellen wirksam. Stärker bevorzugt bei mindestens 80% und am meisten bevorzugt bei mindestens 98% der Zielzellen. Es ist außerdem bevorzugt, dass das Arzneimittel bei höchstens 10% der Kontrollzellen wirksam ist, stärker bevorzugt bei höchstens 5% und am meisten bevorzugt bei < 1% der Kontrollzellen.

Der Begriff "Identifikation von Ribonukleinsäure-Molekülen, deren Expression sich in einer Zielzelle im Vergleich zur Expression einer Kontrollzelle unterscheidet" umfasst in der vorliegenden Erfindung folgende Punkte:
i) Zielzellen sind Zellen in Geweben und Organen, die bekannterweise zur Entstehung einer Krankheit führen, dazu beitragen oder diese verstärken, die die Krankheit aufrecht erhaltenden Prozesse unterhalten, zu ihnen beitragen oder dise verstärken, beziehungsweise die zu Spätfolgen einer Krankheit führen, beitragen oder sie verstärken. Dazu zählen beispielsweise Zellen, die bestimmte Transkriptionsfaktoren aufweisen, spezifische Hormone, Zytokine und Wachstumsfaktoren sezernieren, oder Zellen mit typischen Oberflächenrezeptoren.
ii) Die Zielzellen können zum Beispiel mittels Technologien isoliert werden, die auf der Bindung spezifischer Antikörper basieren. Hier werden Magnetic Beads, erhältlich von den Firmen Miltenyi (Macs-System), Dynal (DynaBeads) oder BD-Bioscience (iMAG) angewendet. Alternativ erfolgt dies über eine Zellreinigung mittels Fluoreszenz-markierter Antikörper an Zellsortern beispielsweise der Firma Cytomation (MOFLO) oder BD-Bioscience (FACS-Vantage). Die Reinheit der Zielzellen ist bevorzugt bei mindestens 80%, stärker bevorzugt bei mindestens 95% und am meisten bevorzugt bei mindestens 99%.
iii) Verfahren zur Isolierung der RNA sind z.B. in Sambrook and Russell, Molecular Cloning, A Laboratory Manual, 3. Auflage, Cold Spring Harbor Laboratory (2001), New York und Ausubel et al., Current Protocols in Molecular Biology, John Wiley & Sons (1998), New York, beschrieben. Außerdem ist es dem Durchschnittsfachmann möglich, kommerziell verfügbare Kits (Silika-Technologie) z.B. das RNeasy Kit von der Firma Qiagen, zur RNA Isolierung zu verwenden. Weiterhin ist es bevorzugt, direkt mRNA aus den Zielzellen durch Verwendung kommerzieller Kits beispielsweise der Firmen Qiagen (Oligotex mRNA Kit), Promega (PolyATract mRNA Isolation System) oder Miltenyi (mRNAdirect) zu reinigen.
iv) Die Identifizierung von mRNAs, die differentiell unterschiedlich sind, d.h. mRNAs, deren Expression in der Zielzelle gegenüber der Kontrollzelle erhöht ist, erfolgt beispielsweise mit kommerziell erworbenen Genchips (z.B. MWG, CLONTECH)] oder mit einem Filter-Hybridisierungsverfahren (z. Bsp. Unigene) nach Herstellerangaben. Alternativ werden differentielle mRNAs durch subtraktive Hybridisierung von cDNA, die zuvor aus der mRNA durch RT-Reaktion entstanden sind, hergestellt. Zu diesen dem Fachmann bekannten Verfahren, zählen beispielsweise die SSH-Methode (Firma Clontech) oder die RDA-Methode. Zu einer ebenfalls bevorzugten Anwendungsform gehört die Kombination von Chiptechnologie und subtraktiver Hybridisierung. Die Identifizierung der differenziell exprimierten Gene erfolgt unter Einsatz der Chiptechnologie mit Hilfe von kommerziell erhältlichen Programmen z.B. mit dem Vector Xpression Programm der Firma InforMax. Beim Einsatz von subtraktiver Hybridisierung erfolgt nach der Isolierung der differentiell exprimierten Gene anhand herkömmlicher, dem Fachmann geläufiger Verfahren wie Klonierung und anschließende Sequenzierung (siehe z.B. Sambrook and Russell, Molecular Cloning, A Laboratory Manual, 3. Auflage, Cold Spring Harbor Laboratory (2001), New York und Ausubel et al., Current Protocols in Molecular Biology, John Wiley & Sons (1998), New York) ein Sequenzabgleich in einer Datenbank wie z.B. Gene-Bank (www.ncbi.nlm.nih.gov).
   Die Expression in der Zielzelle unterscheidet sich im Vergleich zur Expression in einer Kontrollzelle. In einer Ausführungsform des erfindungsgemäßen Verfahrens ist die Expression in der Zielzelle im Vergleich zur Expression in einer Kontrollzelle erhöht, vorzugsweise mindestens um einen Faktor 1,5. In einer besonders bevorzugten Ausführungsform ist die Expression in der Zielzelle im Vergleich zur Expression in einer Kontrollzelle um mindestens einen Faktor 5 erhöht, und in einer am meisten bevorzugten Ausführungsform ist die Expression nur in der Zielzelle, jedoch nicht in der Kontrollzelle nachweisbar.

Der Begriff "Design von Ribonukleinsäure Molekülen, die an Ribonukleinsäure Moleküle aus Schritt a) binden und sie funktionell inaktivieren" umfasst im Sinne der vorliegenden Erfindung die Verwendung von RNA-inaktivierenden-DNA-Enzymen (DNAzymen) und/oder Small-Interfering-RNA (siRNA), die Ribonukleinsäure Moleküle funktionell inaktivieren.
Der Begriff DNAzyme umfasst dabei erfindungsgemäß DNA-Moleküle, die die ZielSequenz der Nukleinsäure, sowohl DNA als auch RNA, spezifisch erkennen und spalten.
Ein generelles DNAzyme-Modell stellt das "10-23"-Modell dar. DNAzyme des 10-23-Modells - auch als "10-23 DNAzyme" bezeichnet - besitzen eine katalytische 25 Domäne von 15 Desoxyribonukleinsäuren, welche von zwei Substratbindungsdomänen flankiert wird. Die Länge der Substratbindungsdomänen ist variabel, sie sind entweder gleich oder unterschiedlich lang. In einer bevorzugten Ausführung, beträgt die Länge der Substratbindungsdomänen zwischen 6 und 14 Nukleotide.
In einer besonders bevorzugten Ausführung sind die Substratbindungsdomänen vollständig komplementär zu der Region, die die Spaltstelle flankiert. Um die Ziel RNA zu binden und sie zu spalten, muss das DNAzyme jedoch nicht unbedingt vollständig komplementär sein. In vitro Untersuchungen zeigen, dass DNAzyme des 10-23 Typs die Ziel mRNA an Purin-Pyrimidin Abfolge-Sequenzen spalten.

Um die DNAzyme in der Behandlung von Krankheiten zu verwenden, ist es bevorzugt, dass die DNAzyme so gut wie möglich gegen Degradation im Körper (im Blut, im intrazellulären Milieu usw.) stabilisiert sind. Eine bevorzugte Ausführung ist die Einführung einer 3'-3'-Inversion an einem oder mehreren Enden des DNA-zymes. Der Begriff 3'-3'-Inversion bezeichnet eine kovalente Phosphatbindung zwischen den 3'-Kohlenstoffen des terminalen Nukleotids und des angrenzenden Nukleotids. Dieser Typ von Bindung steht im Gegensatz zu der normalen Phosphatbindung zwischen den 3' und 5' Kohlenstoffen von aufeinander folgenden Nukleotiden. Dementsprechend wird bevorzugt, dass das Nukleotid am 3'-Ende der an das 3'-Ende der katalytischen Domäne angrenzenden Substratbindungsdomäne invers ist. Zusätzlich zu den Inversionen können die DNAzyme modifizierte Nukleotide oder Nukleotid-Verbindungen enthalten. Modifizierte Nukleotide beinhalten z.B. N3'-P5'-Phosphoramidat Verbindungen, 2'-O-Methyl-Substitutionen und Peptid-Nukleinsäure-Verbindungen. Ihre Herstellung ist dem Fachmann geläufig.

Obwohl die potentiellen DNAzyme-Schnittstellen ubiquitär vorkommen, sind diese oft durch die sekundäre Struktur der RNA blockiert und somit den DNAzymen unzugänglich. Daher werden aus einem Pool an DNAzymen diejenigen selektioniert, deren Schnittstellen frei zugänglich sind. Diese selektionierten DNAzyme sind aktiv, spalten die Ziel-mRNA und inaktivieren sie somit funktionell. Die Effizienz der Spaltung der mRNA durch die einzelnen DNAzyme wird entweder durch Einzeltestung jedes DNAzymes oder durch gekoppelte Testung mehrerer DNAzyme in "Multiplex-Assays" (beschrieben z. B. in Cairns et al., 1999) gezeigt.

Der Begriff siRNA umfasst erfindungsgemäß doppelsträngige, 21-23 Basen lange RNA-Moleküle, die zu einer spezifischen Degradation der komplementären Ziel mRNAs sowohl in vitro als auch in vivo führen. Es ist dem Fachmann anhand der Literatur (z.B. http://www.mpibpc.gwdg.de/abteilungen/100/105/index.html) bekannt, ausgehend von der Ziel-mRNA Sequenz siRNA-Moleküle herzustellen.

Die Wahrscheinlichkeit, dass sich unter drei ausgewählten siRNA-Molekülen mindestens ein hochaktives (Inhibition der Ziel-RNA um mindestens 80%) befindet wird in der Literatur mit mindestens 70% angegeben. Es werden aus einem Pool an siRNA-Molekülen diejenigen selektioniert, die zu einer spezifischen Degeneration der komplementären Ziel-mRNA sowohl in vitro als auch in vivo führen.

Der Begriff "Einbringen der spezifischen Ribonukleinsäure Moleküle aus Schritt b) in Zielzellen" umfasst im Sinne der vorliegenden Erfindung die Transfektion von Vektoren, insbesondere Plasmide, Cosmide, Viren oder Bakteriophagen, die die oben beschriebenen erfindungsgemäßen spezifischen Ribonukleinsäuremoleküle enthalten, in die Zielzellen. Vorzugsweise sind die Vektoren zur Transformation tierischer und humaner Zellen geeignet und erlauben die Integration der erfindungsgemäßen Ribonukleinsäuremoleküle. Verfahren zur Transfektion wie z.B. Lipofektion mittels DMRIE-C der Firma Invitrogen sind dem Fachmann aus der Literatur bekannt. Grundsätzlich sind dafür auch liposomale Vektoren geeignet. Die Zielmoleküle sind Transkriptionsfaktoren, Zellen, die Hormone, Zytokine und Wachstumsfaktoren sezernieren, aber auch Zellen, die auf der Oberfläche der exprimierte Rezeptoren tragen.
Als Kontrollzellen im Sinne der Erfindung werden gesunde Zellen des Zielgewebes, typgleiche Zellen aus anderen Kompartimenten desselben Patienten oder auch aus gesunden Individuen herangezogen.

Die Kultivierung der Zielzelle erfolgt in Nährmedien, die den Bedürfnissen der Zielzelle nach pH-Wert, Temperatur, Salzkonzentration, Antibiotika, Vitaminen, Spurenelementen und Belüftung entsprechend angepasst sind.
Der Begriff Patient bezieht sich gleichermaßen auf Menschen und Wirbeltiere. Damit kann das Arzneimittel in der Human- und Veterinärmedizin verwendet werden.

Der Begriff "Formulierung der spezifischen Ribonukleinsäure Moleküle aus Schritt b) oder einer Zielzelle aus Schritt c) in einem Arzneimittel" umfasst pharmazeutisch akzeptable Kompositionen, die Modifikationen und "Prodrugs" beinhalten, sofern sie nach zuverlässiger medizinischer Beurteilung keine übermäßige Toxizität, Irritationen oder allergische Reaktionen am Patienten auslösen. Der Terminus "Prodrug" bezieht sich auf Verbindungen, die zur Verbesserung der Aufnahme transformiert werden, wie beispielsweise durch Hydrolyse im Blut.

Bevorzugter weise ermöglicht die Formulierung, dass die spezifischen Ribonukleinsäure Moleküle den Patienten in Form einer pharmazeutisch akzeptablen Komposition entweder oral, rektal, parenteral, intravenös, intramuskulär oder subkutan, Intracisternal, intravaginal, intraperitoneal, intrathekal, intravasculär, lokal (Puder, Salbe oder Tropfen) oder in Sprayform verabreicht werden.

Dosierungsformen für die örtliche Administration des Arzneimittels dieser Erfindung schließen Salben, Puder, Sprays oder Inhalationsmittel ein. Die aktive Komponente wird unter sterilen Bedingungen mit einem physiologisch akzeptablen Trägerstoff und möglichen Preservativen, Puffern oder Treibmitteln, je nach Bedarf, vermischt.
Die Art der Dosierung wird vom behandelnden Arzt entsprechend den klinischen Faktoren bestimmt. Es ist dem Fachmann bekannt, dass die Art der Dosierung von verschiedenen Faktoren wie z.B. Körpergröße, Gewicht, Körperoberfläche, Alter, Geschlecht oder der allgemeinen Gesundheit des Patienten abhängig ist, aber auch von dem speziell zu verabreichenden Mittel, der Dauer und Art der Verabreichung und von anderen Medikamenten, die möglicherweise parallel verabreicht werden.

Das mit dem erfindungsgemäßen Verfahren hergestellte Arzneimittel weist eine hohe Patienten-, Krankheits-, Stadien- bzw. Phasenspezifität auf. Es bewirkt eine Zell-spezifische Intervention und ist spezifisch für Kompartimente und Organe. Es entstehen keine oder nur sehr geringe Reaktionen des Immunsystems gegen das Arzneimittel, und das Nebenwirkungsprofil steht in einem akzeptablen Verhältnis zu Schweregrad, Prognose und Verlauf der Erkrankung.
Das Arzneimittel kann zur Therapie gegen sämtliche Erkrankungsgruppen, die mit chronischen Entzündungen einhergehen, wie z.B. Autoimmunerkrankungen, Erkrankungen des rheumatischen Formenkreises (Manifestationen an u. a. Haut, Lunge, Niere, Gefäßsystem, Nervensystem, Bindegewebe, Bewegungsapparat, Endokrinem System), Allergische Soforttypreaktionen und Asthma, Chronisch-obstruktive Lungenerkrankungen (COPD), Arteriosklerose, Psoriasis und Kontaktekzem sowie gegen chronische Abstoßungsreaktionen nach Organ- und Knochenmarkstransplantation angewendet werden.

### Beispiel

### a) Identifikation von Ribonukleinsäure Molekülen, deren Expression in einer Zielzelle sich im Vergleich zur Expression einer Kontrollzelle unterscheidet

i) Als Zielzellen werden die für die Entstehung von chronischen Entzündungsreaktionen verantwortlichen naiven CD4⁺ Zellen verwendet.
ii) Die CD4⁺ Zielzellen werden über Magnetic Beads (Firma Miltenyi (Macs-System) Dynal (DynaBeads) oder BD-Bioscience (iMAG) isoliert, alternativ mittels Fluoreszenz-markierter Antikörper an Zellsortern beispielsweise der Firmen Cytomation (MOFLO) oder BD-Bioscience (FACS-Vantage).
iii) Isolierung der RNA erfolgt nach Standard-Methode, siehe Sambrook.and Russell, Molecular Cloning, A Laboratory Manual, 3. Auflage, Cold Spring Harbor Laboratory (2001), New York und Ausubel et al., Current Protocols in Molecular Biology, John Wiley & Sons (1998), New York.
   Alternativ wird ein RNeasy Kit der Firma Qiagen verwendet, oder es erfolgt direkte Isolierung der mRNA aus CD4⁺Zielzellen mit Oligotex mRNA Kit der Firmen Qia-20 gen nach Herstellerangaben.
iv) Die Identifizierung von mRNAs, die differentiell unterschiedlich sind, d.h. mRNAs, deren Expression in der Zielzelle gegenüber der Kontrollzelle erhöht ist, erfolgt mittels Genchips (z.B. MWG, CLONTECH)], und die Identifizierung der differenziell exprimierten Gene mittels Vector Xpression Programm der Firma Infor-Max.
   Filter-Hybridisierungsverfahren (z. Bsp. Unigene) nach Herstellerangaben. Der Isolierung der differenziell exprimierten Gene schließt sich Klonierung, Sequenzierung (nach Standardvorschriften siehe z.B. Sambrook and Russell, Molecular Cloning, A Laboratory Manual, 3. Auflage, Cold Spring Harbor Laboratory (2001) und der Sequenzabgleich in der Gene-Datenbank (www.ncbi.nlm.nih.gov) an.
   Die Expression von T-bet unterscheidet sich in der Zielzelle (Th1-Zelle) im Vergleich zur Expression in einer Kontrollzelle (beispielsweise Th0-Zelle).

### b) Design von spezifischen Ribonukleinsäure Molekülen, die an Ribonukleinsäure Moleküle aus Schritt a) binden und sie funktionell inaktivieren

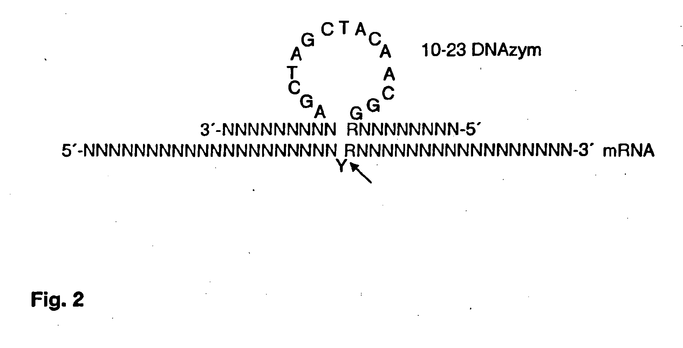

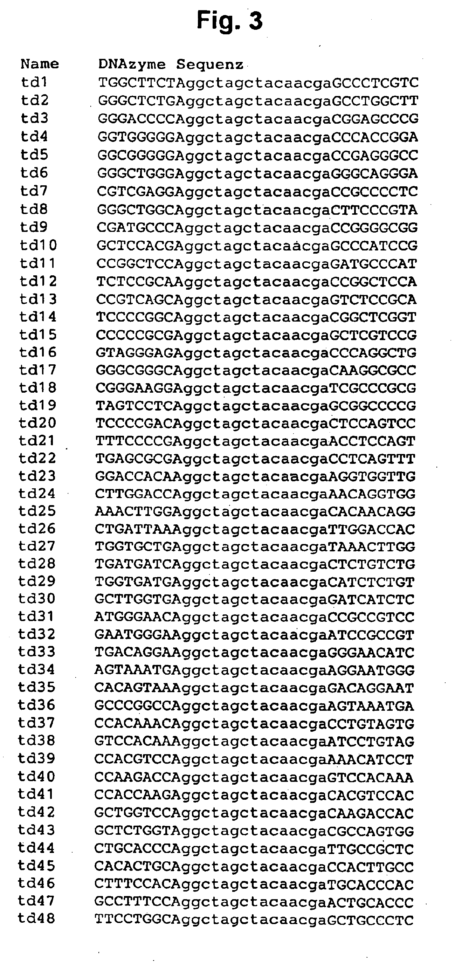

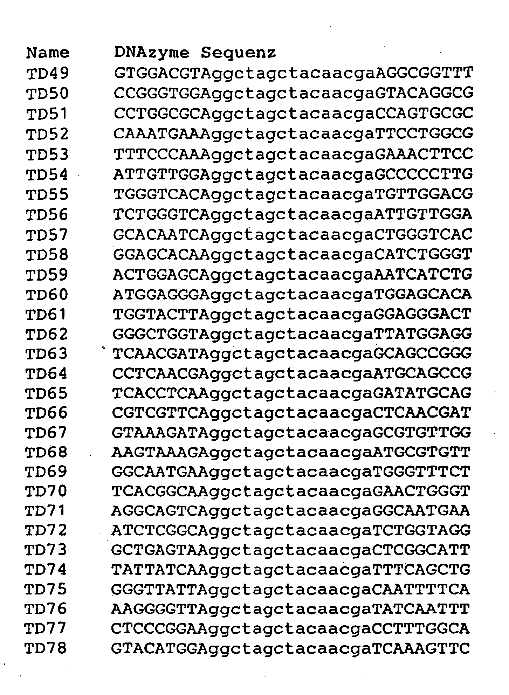

Figur 3 zeigt den erfindungsgemäßen Pool td1-td78 an spezifischen DNAzymen gegen T-bet mRNA. Die DNAzyme weisen eine Gesamtlänge von 33 Nukleotiden auf, wobei die zentrale katalytische Domäne aus 15 Nukleotiden (in kleingeschriebenen Buchstaben) der katalytischen Domäne des bekannten 10-23 DNAzyme (Figur 2) entspricht. Diese katalytische Domäne wird von zwei, aus jeweils 9 Nukleotiden bestehenden rechten und linken Substratbindungsdomäne (in großgeschriebenen Buchstaben) flankiert. Die Nukleotidsequenz der rechten und linken Substratbindungsdomäne ist unterschiedlich und variiert bei den DNAzymen td1 bis td78, so dass eine unterschiedlich spezifische Bindung mittels Watson-Crick Paarung an die T-bet mRNA erfolgt.

Figur 2 zeigt das allgemeine Modell zur Bindung des 10-23 DNAzyme an eine mit N markierte beliebige Ziel RNA, wobei der Pfeil auf die Spaltstelle in der Ziel mRNA hinweist.

Da aus der Literatur bekannt ist, dass DNAzyme die Ziel-mRNA an Purin-Uracil-Bindungen effektiver spalten als Purin-Cytosin-Bindungen, werden vorzugsweise 5 DNAzyme konstruiert, die an Purin-Uracil-Bindungen spalten.
Das in Figur 2 gezeigte Modell kann in seiner Funktionsweise auf die Bindung der DNAzyme td1 bis td78 an T-bet mRNA übertragen werden.
Die DNAzyme td1 bis td78 werden für in vitro Versuche unmodifiziert eingesetzt, für Versuche in Zellkultur mit Modifikationen versehen (käuflich erworben durch Firma Eurogentec).
Als Modifikationen zur Stabilisierung und Schutz werden eingesetzt:
1) Ein stabilisierendes inverses Thymidin am 3'-Ende
2) eine FAM-Markierung am 5'-Ende zur Beurteilung der Transfektionseffizienz der Zellen mittels FACS-Analyse.

Zur Darstellung der Spaltungseigenschaften der DNAzyme und funktionellen Inaktivierung der Ziel mRNA der T-bet mRNA erfolgt in vitro Transkription der T-bet-mRNA aus humanem EDTA Vollblut mittels QlAamp-RNA-Blood-Mini-Kit (Qiagen, Deutschland) nach Herstellerangaben.
Figur 4 zeigt die Nukleotidsequenz von humanem T-bet, wie es der Datenbankeinträgen [PubMed (http://www.ncbi.nlm.nih.gov/entrez/query. fcgi?db=Nucleotide)] Nr.: NM_013351, Sequenz 1 zu entnehmen ist.
Die reverse Transkription erfolgt mit dem Forward-Primer CGGCCCGCTGGA-GAGGAAGC und Reverse-Primer CACACACCCACACACAACC nach Standardvorschrift (ThermoScript von Invitrogen), wobei ein PCR-Produkt mit einer Länge von 2450 Nukleotiden amplifiziert wird. Dieses PCR-Produkt wird mittels Standardverfahren in das Plasmid pBluescript-SK (Stratagene) kloniert und zur Überprüfung sequenziert.
Figur 4 zeigt einen Vergleich der Nukleinsäuresequenz von T-bet Nr.: NM_013351 (Sequenz 1) und sequenzierter Sequenz (Sequenz 2). Dabei zeigt sich das beide Sequenzen nicht vollkommen identisch sind, sondern einzelne Basen ausgetauscht sind. Die Nukleinsäuresequenz 2 von T-bet aus Figur 4 bildet in dieser Erfindung die Grundlage für die Konstruktion von DNAzymen gegen T-bet mRNA.

Figur 4A zeigt die Nukleotidsequenz der Sequenz 1 des humanen T-bet-Gen aus Figur 4 und darin als graue Hinterlegung eingezeichnet jeweils zwei Nukleotide GT bzw. AT, zwischen denen weitere potentielle DNAzym-Schnittstellen liegen.

Die Herstellung von T-bet mRNA erfolgt nach Linearisierung des T-bet enthaltenden Plasmids pBluescript-SK durch Spaltung mit dem Restriktionsenzym Xba I (Fermentas) und durch in-vitro-Transkription nach Herstellerangaben (Ambion). T-bet mRNA liegt mit einer Länge von insgesamt 2550 Nukleotiden vor.

Die in vitro Spaltungsexperimente von T-bet mRNA mit den DNAzymen (td1 bis td78) werden in einem Volumen von 10 µl folgender Reaktionszusammensetzung durchgeführt: 50 mM Tris pH 7,4, 150 mM NaCl, 10 mM MgCl2, 0,25 µM DNAzyme und 0,025 µM in vitro transkribierte GATA-3 mRNA (in einem Substrat zu DNAzyme Verhältnis von 1:10). Die Reaktionen werden bei 37°C für die jeweils angegebenen Zeiten inkubiert. Durch die Zugabe von Formamid- und EDTA-haltigem RNA-Sample-Loading-Buffer (Sigma) wird die Reaktion gestoppt. Die denaturierten Proben werden in 1,3 %igen TAE-Agarose-Gelen aufgetrennt und im UV-Transilluminator analysiert.

Figur 5 zeigt als Ergebnis der Gelelektrophorese die Spaltung der T-bet Ziel-mRNA mit modifizierten DNAzymen [td54-M (Spur 3), td69-M (Spur 4), td70-M (Spur 5)]. Spur 2 enthält als Kontrolle T-bet Ziel-mRNA ohne DNAzym-Zugabe. Ein mitgeführter Längenstandard (Spur M) zeigt Bandengrößen von 2000 bp und 3000 bp. Pfeile zeigen auf A, die Bande mit dem Substrat (hier T-bet mRNA) und auf B eines der beiden Spaltprodukte (das andere Spaltprodukt ist auf dieser Abbildung nicht zu sehen).

Der Vergleich zwischen allen 78 DNAzymen zeigt, dass td54, td69 und td70 besonders aktiv sind, die Modifikationen die Effektivität der DNAzyme nicht herabsetzen.
Die folgende Tabelle zeigt die Einteilung der DNAzyme td 1 bis td 78 gegen t-bet 3 mRNA in 4 Gruppen. Diese Gruppeneinteilung erfolgt aufgrund durchgeführter in-vitro Aktivitätstestungen der DNAzyme gegen t-bet mRNA. Gruppe 1: hohe Spaltungsaktivität, Gruppe 2: mittlere Spaltungsaktivität, Gruppe 3: schwache Spaltungsaktivität und Gruppe 4: keine messbare Spaltungsaktivität.

| **Gruppe** | **td** | **Aktivität gegen t-bet mRNA** |
|---|---|---|
| **1** | 54, 69, 70 | Hohe Spaltungsaktivität |
| **2** | 21, 24, 28, 29, 30, 45, 71, 72, 77, 78 | Mittlere Spaltungsaktivität |
| **3** | 13, 19, 22, 23, 25, 27, 31, 32, 44, 46,47, 48, 50, 51, 53, 55, 56, 57, 58,60,61,62, 65, 67,68, 73,74,75 | Schwache Spaltungsaktivität |
| **4** | 1,2,3,4,5,6,7,8,9,10,11,12,14,15,16,17,18, 20, 26, 33, 34, 35, 36, 37, 38, 39, 40, 41, | Keine Spaltungsaktivität |

### c) Einbringen der spezifischen Ribonukleinsäure Moleküle aus Schritt b) in Zielzellen

Die DNAzyme td54, td69 und td70 werden mit und ohne die beschriebenen Modifikationen in Zielzellen verwendet.

Dazu werden Jurkat E6.1 Zellen (human acute T cell leukemia Cells) im RPMI-Medium mit 100 U/ml Penicillin, 0,1 mg/ml Streptomycin und 10% FKS bei 37°C in befeuchteter 5 %iger CO2-Atmosphäre kultiviert. Die Transfektionen werden in 6-Wellplatten durchgeführt. Hierfür werden 2x10⁶ Jurkat E6.1 Zellen in Opti-MEM-I-Zellkulturmedium (Invitrogen) überführt und mittels DMRIE-C (Invitrogen) mit den modifizierten DNAzymen (0,3 µM) transfiziert (nach Herstellerangaben der Firma Invitrogen). Nach 10 Stunden Inkubation im Brutschrank unter obigen Bedingungen wird RPMI-Medium (mit den oben angegebenen Zusätzen) hinzu gegeben und die Inkubation für weitere 14 Stunden fortgesetzt. Die Zellen werden mit Opti-MEM-Medium gewaschen und anschließend erneut nach dem oben beschriebenen Protokoll transfiziert. Nach jeder Transfektion wird die Transfektionseffizienz mittels FACS-Analyse beurteilt.

Nach der Transfektion von Jurkat E6.1 Zellen wird die T-bet-mRNA Menge relativ zu GAPDH-mRNA Expression mittels Real-Time-PCR (LightCycler, Roche) quantitativ bestimmt, um Aussagen über die in vitro Effektivität der DNAzyme zu erhalten.

Für LightCycler-Analysen wird die RNA aus den Jurkat E6.1 Zellen mittels RNeasy Mini Kit (Qiagen, Deutschland) gereinigt und nachfolgend photometrisch normlaisiert. Nach reverser Transkription mit SuperScript II (Gibco) laut Herstellerangaben, folgt die quantitative Analyse der T-bet und GAPDH-mRNA im LightCycler. Das Gesamtvolumen für die PCR ist 20 µl, darin enthalten sind 1 µl DNA, je 1 µl (0,5 µM) Sense- und Antisense-Primer sowie 10 µl QuantiTect-SYBR-Green-PCR-Master-Mix (Qiagen, Deutschland). Die verwendeten PCR-Primer für T-bet sind: Sense 5'-CCCACCATGTCCTACTACCG-3'; Antisense 5'-GCAATCTCAGTCCACACCAA-3'. Die PCR-Primer für GAPDH sind: Sense 5'-TCTTCTTTTGCGTCGCCAG-3' und Antisense 5'-AGCCCCAGCCTTCTCCA-3'. Die PCR Konditionen sind: Denaturierung (15 min 95°C), Amplifikation (15 sec 95°C, 25 sec 59°C, 25 sec 72°C von 50 Zyklen) dann Final-Extention 2 min. 72°C. Die anschließende Schmelzkurve wird folgendermaßen generiert: 0 sec 95°C, 15 sec 60°C dann wird die Temperatur in 0,2°C Schritten erhöht auf 97°C, gleichzeitig wird kontinuierlich die Fluoreszenz gemessen. Die Schmelzkurve dient der internen Kontrolle, da alle PCR-Produkte eine spezifische Schmelztemperatur haben.

SYBR-Green ist ein Fluoreszenzfarbstoff (enthalten im QuantiTect SYBR Green PCR Master Mix), der an doppelsträngige DNA bindet. Wenn während der Extension die DNA verdoppelt wird, bindet SYBR-Green daran und generiert ein bindungsabhängiges Fluoreszenzsignal, welches von LightCycler am Ende jeder Extension detektiert wird. Je höher die Menge an Ausgangsmaterial, desto früher wird die signifikante Erhöhung der Fluoreszenz detektiert. Die LightCycler Software stellt gesammelte Fluoreszenz-Intensitäten gegen die Zyklen graphisch dar.

In der Fig. 6 sind T-bet- und GAPDH-mRNA LightCycler Amplifikationskurven nach der Behandlung von Jurkat E6.1 Zellen mit den DNAzyme td54m, td69m und td70m im Vergleich zu Nonsens-DNAzym behandelten, dargestellt.

Der jeweilige "Crossing Point" (Ct), definiert als der PCR-Zyklus, bei dem sich die Fluoreszenz zum erstmal signifikant von der Hintergrund-Fluoreszenz unterscheidet, wird manuell mit der Fit-Point-Methode der LightCycler Software bestimmt. Die relative Quantifizierung von T-bet- und GAPDH-mRNA in mit DNAzymen behandelten Zellen im Vergleich zu Nonsense-DNAzym behandelten Zellen wird nach der im User Bulletin #2 (ABI Prism 7700 Sequence detection System User Bulletin #2 (2001) Relative quantification of gene expression http://docs.appliedbiosystems.com/pebiodocs/04303859.pdQf) beschriebenen Anleitung durchgeführt. Dabei werden die Menge an T-bet-mRNA aus dem Kontrollversuch gleich 100% gesetzt. Die Daten der relativen Quantifizierung sind in der Fig. 7 graphisch dargestellt.

Im Vergleich zur Nonsense-DNAzym Behandlung zeigt sich, dass das td69m-DNAzyme zu einer Suppression von 81,3% und das td70m-DNAzyme zu einer Suppression von 81,0% führt, wohingegen das td54m-DNAzym keinen suppressiven Effekt auf T-bet-mRNA hat.

Das bedeutet, dass das td54m-DNAzym in vivo nicht aktiv ist, wohingegen td69mund td70m-DNAzyme auch im zellulären Milieu die mRNA von T-bet inaktivieren. Die spezifische Reduktion der T-bet mRNA in vivo durch die DNAzyme td69m und td70m stellt somit ein effektives therapeutisches Werkzeug zur Behandlung von chronisch entzündlichen Erkrankungen dar.

### d) Formulierung der spezifischen Ribonukleinsäure aus Schritt b) und/oder einer Zielzelle aus Schritt c) in einem Arzneimittel

Die Analyse verschiedener DNAzyme mit für T-bet spezifischer Substratbindedomäne zeigt, dass DNAzyme td69 und td70 die T-bet Expression in vivo spezifisch inhibieren und als spezifische Ribonukleinsäure zur Herstellung eines Zell- und/oder Gewebe- und/oder Krankheitsphasen-spezifischen Arzneimittels geeignet sind.

Dazu wird td69 (GGCAATGAAggctagctacaacgaTGGGTTTCT) oder td70 (TCACGGCAAggctagctacaacgaGAACTGGGT) oder mit td69m bzw. td70m transfizierte Zellen in einer pharmazeutischen Komposition mit einem pharmazeutisch akzeptablen Carrier beispielsweise Liposome oder bioabbaubare Polymere versehen.

Alternativ zu den DNAzymen wird zur spezifischen Inhibition der T-bet Expression und zur Herstellung eines Zell- und/oder Gewebe- und/oder Krankheitsphasenspezifischen Arzneimittels der Einsatz von siRNA vorgeschlagen.

Vorzugsweise handelt es sich um siRNA zur Inhibition von humanem T-bet. Die Herstellung der siRNA ist dem Fachmann bekannt und in der Literatur beschrieben. Ein Beispiel für siRNA Sequenzen:

| **Quelle** | **Nukleinsäuresequenzen** |
|---|---|
| Human T-bet | Sense-Strang: UCAGCACCAGACAGAGAUGdTdT |
| | Antisense Strang: CAUCUCUGUCUGGUGCUGAdTdT |

Dem Fachmann ist ersichtlich, dass mit dem Wissen der vorliegenden Erfindung auch leicht spezifische DNAzyme bzw. siRNAs als Arzneimittel bei chronisch entzündlichen Erkrankungen und Autoimmunerkrankungen herstellbar sind, die gegen weitere Transkriptionsfaktoren gerichtet sind, die eine Rolle bei der Differenzierung zu TH1- beziehungsweise TH2-Zellen spielen beispielsweise STAT4, STAT5a, STAT1, c-Rel, CREB2, ATF-2, ATF-2, Hlx, IRF-1, c-Maf, NFAT, NIP45, AP1, Mel-18, SKAT-2, CTLA-4 oder die gegen weitere Faktoren der Signaltransduktionswege zur Differenzierung und/oder Expression von Zytokinen gerichtet sind, beispielsweise Src kinase, Tec kinase, Rlk (Txk im Menschen), Itk, Tec, RIBP, PLCγ, MAP kinase, ERK, JNK, P38, MKK, MKK1, MKK2, MKK3, MKK4, MKK6, MKK7, Rac2, GADD45, GADD45β, GADD45y, SOCS, CIS, SOCS1, SOCS2, SOCS3, JAK, JAK1, JAK3, NIP45.

Diese Proteine weisen eine Expression auf, die in einer Zielzelle im Vergleich zur Expression einer Kontrollzelle erhöht ist

## Patentansprüche

1. DNAzyme, **dadurch gekennzeichnet, dass** sie bestehen aus
- einer katalytischen Domäne mit der Nukleotidsequenz GGCTAGCTACAACGA oder einer modifizierten Sequenz mit vergleichbarer biologischer Wirkung, die die T-bet mRNA an jeder Purin-Pyrimidin Bindungsstelle schneidet, an der sie gebunden ist,
- einer rechten Substratbindedomäne, die sich am 3'-Ende der katalytischen Domäne anschließt und
- einer linken Substratbindedomäne, die sich am 5'-Ende der katalytischen Domäne anschließt, wobei die beiden Substratbindedomänen jeweils komplementär zu zwei Regionen der T-bet-mRNA sind, so dass sie mit der mRNA hybridisieren,
- in vivo aktiv sind und
- die Sequenz td69 GGCAATGAA GGCTAGCTACAACGA TGGGTTTCT oder td70 TCACGGCAA GGCTAGCTACAACGA GAACTGGGT enthalten.

2. DNAzyme nach Anspruch 1, **dadurch gekennzeichnet, dass** sie die katalytische Domäne der T-bet mRNA an einer Purin-Uracil Bindungsstelle schneiden.

3. DNAzyme nach den Ansprüchen 1 und 2, **dadurch gekennzeichnet, dass** sie gegen den Abbau im Organismus durch die Einführung einer 3'-3' Inversion stabilisiert sind.

4. DNAzyme nach den Ansprüchen 1 bis 3, **dadurch gekennzeichnet, dass** sie gegen den Abbau im Organismus durch die Einführung von modifizierten Nukleotiden oder Nukleotidverbindungen stabilisiert sind.

5. DNAzyme nach den Ansprüchen 1 bis 4, **dadurch gekennzeichnet, dass** sie als Modifikation ein inverses Thymidin am 3'-Ende und/oder eine FAM-Markierung am 5'-Ende aufweisen.

6. Verfahren zur Herstellung eines Arzneimittels zur Behandlung von chronischen Entzündungen, **dadurch gekennzeichnet, dass** man
- Zielzellen ermittelt, in denen die Expression von T-bet mRNA höher ist als in gesunden Körperzellen,
- in vivo wirksame DNAzyme gemäß Anspruch 1 entwickelt, die an T-bet mRNA binden und sie funktionell inaktivieren,
- die in vivo wirksamen DNAzyme in Zielzellen einbringt und
- Arzneimittel formuliert, die die in vivo wirksamen DNAzyme enthalten.

7. Arzneimittel enthaltend ein DNAzym gemäß den Ansprüchen 2 bis 5 und einen pharmazeutisch akzeptablen Carrier.

8. Verwendung eines DNAzyms gemäß einem oder mehreren der Ansprüche 1 bis 5 zur Herstellung eines Arzneimittels zur Behandlung von chronischen Entzündungen.

9. Verwendung eines DNAzyms gemäß einem oder mehreren der Ansprüche 1 bis 5 zur Herstellung eines Arzneimittels zur spezifischen Inhibition der T-bet Expression in Zielzellen von Patienten **zur Behandlung von chronischen Entzündungen.**

## Claims

1. DNAzymes, **characterized in that** they consist of:
- a catalytic domain with nucleotide sequence GGCTAGCTACAACGA or a modified sequence of comparable biological effect, which cleaves T-bet mRNA at every purine-pyrimidine binding site to which it is bound;
- a right substrate binding domain which is contiguous with the 3' end of the catalytic domain; and
- a left substrate binding domain which is contiguous with the 5' end of the catalytic domain, whereby the two substrate binding domains are respectively complementary to two regions of the T-bet mRNA so that they hybridize with the mRNA;
- are active in vivo; and
- contain the sequence td69 GGCAATGAA GGCTAGCTACAACGA TGGGTTTCT or td70 TCACGGCAA GGCTAGCTACAACGA GAACTGGGT.

2. DNAzymes according to claim 1, **characterized in that** they cleave the catalytic domain of the T-bet mRNA at a purine-uracil binding site.

3. DNAzymes according to claims 1 and 2, **characterized in that** they are stabilized against degradation in the organism by the introduction of a 3'-3' inversion.

4. DNAzymes according to claims 1 to 3, **characterized in that** they are stabilized against degradation in the organism by the introduction of modified nucleotides or nucleotide compounds.

5. DNAzymes according to claims 1 to 4, **characterized in that** they exhibit, as a modification, an inverse thymidine at the 3' end and/or a FAM-tag at the 5' end.

6. A method for manufacturing a medication for the treatment of chronic inflammations, **characterized in that**:
- target cells are detected in which the expression of T-bet mRNA is higher than in healthy cells of the body;
- DNAzymes according to claim 1 which are active in vivo are developed which bind to T-bet mRNA and functionally inactivate it;
- the DNAzymes which are active in vivo are introduced into target cells; and
- medication is formulated which contains the DNAzymes which are active in vivo.

7. A medication containing a DNAzyme according to claims 2 to 5 and a pharmaceutically acceptable carrier.

8. Use of a DNAzyme according to one or more of claims 1 to 5, for the manufacture of a medication for the treatment of chronic inflammations.

9. Use of a DNAzyme according to one or more of claims 1 to 5, for the manufacture of a medication for the specific inhibition of T-bet expression in target cells from patients for the treatment of chronic inflammations.

## Revendications

1. Désoxyribozymes, **caractérisées en ce qu'**elles sont constituées
- d'un domaine catalytique comportant la séquence de nucléotides GGCTAGCTACAACGA ou une séquence modifiée exerçant un effet biologique comparable qui coupe l'ARNm de T-bet à chaque site de liaison purine-pyrimidine auquel il est lié,
- d'un domaine de liaison au substrat dit de droite qui se situe dans la suite du domaine catalytique vers l'extrémité 3' et
- d'un domaine de liaison au substrat dit de gauche qui se situe dans la suite du domaine catalytique vers l'extrémité 5', les deux domaines de liaison au substrat étant chacun complémentaire à deux régions de l'ARNm de T-bet, permettant ainsi leur hybridation avec l'ARNm,
- et qu'elles présentent une activité in-vivo et
- qu'elles contiennent la séquence td69 GGCAATGAA GGCTAGCTACAACGA TGGGTTTCT ou td70 TCACGGCAA GGCTAGCTACAACGA GAACTGGGT.

2. Désoxyribozymes selon la revendication 1, **caractérisées en ce qu'**elles coupent le domaine catalytique d'ARNm de T-bet à un site de liaison purine-uracile.

3. Désoxyribozymes selon les revendications 1 et 2, **caractérisées en ce qu'**elles sont stabilisées contre leur dégradation dans l'organisme par l'introduction d'une inversion 3'-3'.

4. Désoxyribozymes selon les revendications 1 à 3, **caractérisées en ce qu'**elles sont stabilisées contre leur dégradation dans l'organisme par l'introduction de nucléotides ou de composés nucléotidiques modifiés.

5. Désoxyribozymes selon les revendications 1 à 4, **caractérisées en ce qu'**elles sont modifiées par une thymidine inversée à leur extrémité 3' et/ou par un marquage FAM à leur extrémité 5'.

6. Procédé de préparation d'un médicament destiné au traitement d'inflammations chroniques, **caractérisé en ce qu'**on
- identifie les cellules cibles dans lesquelles le niveau d'expression de l'ARNm de T-bet est plus élevé que dans des cellules saines du corps,
- développe des désoxyribozymes selon la revendication qui présentent une activité in-vivo et qui entrent en liaison avec l'ARNm de T-bet, inactivant ainsi les fonctions de ce dernier,
- introduit dans les cellules cibles les désoxyribozymes présentant une activité in-vivo et
- formule des médicaments contenant les désoxyribozymes présentant une activité in-vivo.

7. Médicament contenant une désoxyribozyme selon les revendications 2 à 5 ainsi qu'un support pharmaceutiquement acceptable.

8. Utilisation d'une désoxyribozyme selon l'une ou plusieurs des revendications 1 à 5 pour préparer un médicament destiné au traitement d'inflammations chroniques.

9. Utilisation d'une désoxyribozyme selon l'une ou plusieurs des revendications 1 à 5 pour préparer un médicament destiné à inhiber spécifiquement l'expression de T-bet dans les cellules cibles de patients, permettant ainsi de traiter des inflammations chroniques.
